(19) Europäisches Patentamt
European Patent Office
Office européen des brevets

(11) EP 4 163 380 A1

(12) EUROPEAN PATENT APPLICATION

(43) Date of publication:
**12.04.2023 Bulletin 2023/15**

(21) Application number: **21201750.3**

(22) Date of filing: **08.10.2021**

(51) International Patent Classification (IPC):
***C12N 15/82*** *(2006.01)* ***G01N 33/543*** *(2006.01)*

(52) Cooperative Patent Classification (CPC):
**G01N 33/543**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **ETH Zurich**
**8092 Zürich (CH)**

(72) Inventors:
- **Leroux, Jean-Christophe**
  **8093 Zurich (CH)**
- **Krivitsky, Adva**
  **8093 Zurich (CH)**
- **Krivitsky, Vadim**
  **8093 Zurich (CH)**

(74) Representative: **Schulz Junghans Patentanwälte PartGmbB**
**Großbeerenstraße 71**
**10963 Berlin (DE)**

# (54) DEVICE AND METHOD FOR MANIPULATION OF EXTRACELLULAR VESICLES

(57) The invention relates to a method for isolating a biomolecule. A liquid sample comprising the biomolecule is contacted with an electrically conductive surface. The surface carries a chemical modification facilitating retention of said biomolecule, or an electrical retention potential is applied to said surface. The biomolecule is released by applying a voltage to the surface.

The invention further relates to a device comprising a chamber configured for receiving a liquid sample, wherein a first surface of said chamber is a working electrode formed by a high-surface, electrochemically active material embedded in a non-electrically conductive polymer matrix. The device further comprises a counter electrode and connections to a voltage source.

The invention further relates to a device and method for loading an extracellular vesicle with cargo molecules.

EP 4 163 380 A1

## Description

The present invention relates to a method and device for separating and/or manipulating biomolecules and, particularly, extracellular vesicles, by attachment of biomolecules to a microstructured, conductive surface. The attached biomolecule may be manipulated and, in certain embodiments, loaded, by applying voltage, and subsequently released by application of a voltage to the surface. The invention relates to a flow cell having a working and counter electrode covered with a ligand or an attachment layer for a biomolecule.

## Background of the Invention

Extracellular vesicles (EV) have emerged as promising therapeutic agents and substitute for cell therapy, due to their advantageous handling and stability, their safety, bioactivity, and low immunogenicity. Additionally, extracellular vesicles, such as exosomes, serve as important biomarkers and are involved in a wide range of important physiological functions. Despite the growing interest in this field, current knowledge is, in part, limited by inefficient isolation techniques. Current methods of isolation include ultracentrifugation, ultrafiltration, and immunoaffinity capturing, time-consuming methods that produce waste and can retain impurities.

Several techniques have been described in the literature for the purification and isolation of EVs from various biosamples. These methods are mainly based on centrifugation, filtration, and affinity capture. Centrifugation and ultracentrifugation, being the most commonly used amongst these methods, rely on multiple centrifugation steps that are tedious and time-consuming. For example, some purifications require up to 5 centrifugation steps that may proceed for several hours. Furthermore, these methods suffer from low specificity towards exosomes as they also spin down larger vesicles and various aggregates. In addition, these methods require specialized facilities. Methods that rely on immune-affinity capture, may eliminate the need for ultracentrifugation. For example, Dynabeads® uses magnetic beads that are coated with antibodies against proteins that are expressed on exosomal membranes. However, this method includes multiple steps, has a low yield, and is not suitable for large samples. In addition, it still requires centrifugation and eluting buffer exchange in order to release the exosomes from the beads (US8901284B2). To eliminate the need for ultracentrifugation and expensive antibody-conjugated beads, methods relying on volume-excluding polymers were suggested. Two poly(ethylene glycol) (PEG)-based products are ExoQuick (U.S. Pat. Appl. 20130337440, System Biosciences, Mountain View, CA, USA) and Total Exosome Isolation Reagent (U.S. Pat. Appl. 20130273544, Life Technologies, Carlsbad, CA, USA). These methods utilize the high water affinity of some polymers for the precipitation of less-soluble components, subsequently collected by short centrifugation. However, contamination with aggregates, macromolecular complexes, and proteins commonly occurs. Furthermore, these methods are time-consuming and still require bulky equipment such as centrifuges. Filtration-based methods for capturing exosomes can be performed, relying on defined pore-size matrices. For example, ExoMir™ uses a series of two filters of different pore-size (US 2013/0052647A1). Alternatively, size exclusion chromatography can be used, employing crosslinked polymers such as dextran (Sephadex), agarose (Sepharose), polyacrylamide (Biogel P), or allyldextran (Sephacryl), as the separating matrix. Nonetheless, these methods are time-consuming, not specific, and may collect all components of similar size, and their yields are inconsistent.

Finally, microfluidics-based techniques that are usually used for microscale isolation, are based on various exosomal properties such as immuno-affinity, size, and density. The main advantages of these techniques are their fast performance, low cost, portability, and their simple automation. However, they suffer from low sample capacity and were not tested on clinical samples on a large scale.

The main hurdle in EV isolation is their sparseness in their source biofluid. Hence, significant volume reduction is essential to obtain sufficient concentration. Thus, there is a need for new and improved methods of capturing, manipulating, and releasing EVs. For this purpose, the development of a device that processes diverse samples (from 0.005-500 mL) of raw biological material without specialized experimental equipment is critical.

Classical electroporation is well established for microorganisms, but still possesses a challenge for loading exosomes. Classical electroporation requires high voltage pulses, that may destroy biological entities as well as the electrodes themselves, retaining impurities. Furthermore, electroporation is hard to integrate on a single platform with purification.

Based on the above-mentioned state of the art, the objective of the present invention is to provide means and methods to separate and isolate molecules, biomolecules, particularly extracellular vesicles such as exosomes, microvesicles, or apoptotic bodies, and load them with membrane-impermeable molecules such as nucleic acids and charged proteins/peptides.

This objective is attained by the subject matter of the independent claims of the present specification, with further advantageous embodiments described in the dependent claims, examples, figures, and general description of this specification.

US10948451B2 is incorporated by reference herein, as is any other patent document mentioned herein.

## Summary of the Invention

In one aspect, the invention relates to a method for isolating a biomolecule. The method comprises contacting a liquid sample comprising said biomolecule with a surface formed by an electrically conductive micro- or nanostructured material. Subsequent to binding of the biomolecule, or of a vesicular structure presenting the biomolecule, the surface is washed. After applying a voltage to said surface, the biomolecule or vesicle is released into a collection solution and collected.

In one particular set of embodiments, the surface comprises a ligand capable of specifically binding to said biomolecule attached thereto.

In another aspect, the invention relates to a method for loading an extracellular vesicle with a cargo molecule. This method comprises the steps of providing an aqueous medium comprising an extracellular vesicle, the aqueous medium being in contact with a surface that is electrically conductive. The aqueous medium is a loading solution that comprises a cargo molecule to be transferred into the extracellular vesicle. A loading voltage is applied to the surface, thereby allowing entry of the cargo molecule into the extracellular vesicle.

The difference to electroporation methods known in the art is that the surface is an electrically conductive micro- or nanostructured material.

In yet another aspect, the invention relates to a device for isolating a biomolecule by the method outlined above. This device comprises a chamber configured for receiving a liquid sample, wherein a first surface of said chamber is formed by a high-surface, electrochemically active (conductive) material embedded in a non-electrically conductive polymer matrix, said electrochemically active material having a ligand capable of specifically binding to said biomolecule attached thereto, said first surface forming a working electrode; and said device comprises a counter electrode configured to contact said liquid sample; and a first and second electrically conductive connection connectable to a voltage source is connected to said working and counter electrode, respectively.

The device of the invention integrates purification and preconcentration in one step, controllably releases the analyte by applying an electric potential, and can perform electrochemical analysis. Alternatively, the device facilitates purification, preconcentration and loading with cargo molecules on a single platform in two subsequent stages, or can act downstream to a separate purification step as a loading platform.

In certain aspects, the invention relates to a novel separation method and device having high throughput capabilities. In certain embodiments, the device is composed of a 3-dimensional micro carbon fiber electrode bearing a high surface area embedded in a polydimethylsiloxane (PDMS)-based fluidic channel. Surface conjugation with ligands (also referred to herein as "affinity agents") against EV surface proteins allows specific and selective separation. The controlled release is driven by electrical voltage. Isolation of large amounts of EVs straight from biological fluids enables thorough investigation into their therapeutic activity. Electroporation of EV with impermeable molecules facilitates the investigation of their properties, and their use as drug delivery vehicles.

As opposed to current isolation methods, the device according to the invention is tailored for processing large volumes (for example 5 $\mu$L - 500 mL) of raw biological samples, within a time frame of few minutes. The device operates in the following mode: a 3D-microstructured carbon electrode with a high surface area is embedded within a PDMS-based molded fluidic channel. Surface modification of the carbon electrode allows conjugation with affinity agents against prevalent EV-specific surface proteins. The EV source/biosample is injected directly into the fluidic channel. Adsorbed EVs are washed and controllably released to the desired medium by applying an electric potential. The expected yield depends on the capacity of the device. For example, a device bearing a 58 mm-long fluidic channel bearing microcarbon-fibers electrodes modified with anti CD9 antibodies, yielded 1-100 $\mu$g of exosomal protein, from 1-200 mL of raw sample.

The purification system and methods disclosed herein meet the requirements of a fast and robust methodology for capturing and release of EVs by being highly selective, enabling flexible sampling volume while maintaining handling simplicity. The purification system disclosed herein is designed as hand-held devices, with minimized and miniaturized dimensions, weight, and costs.

The device disclosed herein allows integration of purification, preconcentration, and loading with cargo molecules on a single platform in two subsequent stages, or can act downstream to a separate purification step as a loading platform. Classical electroporation is well established for microorganisms, but still possesses a challenge for loading exosomes. Classical electroporation requires high voltage pulses that may destroy biological entities as well as the electrodes themselves, leaving impurities. Furthermore, electroporation is hard to integrate on a single platform with purification. The device presented herein utilizes low voltage that allows maintaining the integrity of biological entities and of the electrodes, which are robust and stable. In addition, it only requires a simple power source, which enables portability. Affinity to the surface allows easy solution exchange and sequential electroporation with several materials, while a separate release step allows to control and select the elution buffer. The cargo molecules can be macromolecules such as oligonucleotides, aptamers, peptides, or small molecules.

*Terms and definitions*

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising," "having," "containing," and "including," and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, a description referring to "about X" includes a description of "X."

As used herein, including in the appended claims, the singular forms "a," "or," and "the" include plural referents unless the context clearly dictates otherwise.

Unless defined otherwise, all technical and scientific terms used herein have the same meaning as commonly understood by one of ordinary skill in the art (e.g., in cell culture, molecular genetics, nucleic acid chemistry, hybridization techniques, and biochemistry). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

*Binding; Binders Ligands Antibodies:*

The term *specific binding* in the context of the present invention refers to a property of ligands that bind to their target with a certain affinity and target specificity. The affinity of such a ligand is indicated by the dissociation constant of the ligand. A specifically reactive ligand has a dissociation constant of $\leq 10^{-7}$ mol/L when binding to its target, but a dissociation constant at least three orders of magnitude higher in its interaction with a molecule having globally similar chemical composition as the target, but a different three-dimensional structure.

In the context of the present specification, the term *dissociation constant ($K_D$)* is used in its meaning known in the art of chemistry and physics; it refers to an equilibrium constant that measures the propensity of a complex composed of [mostly two] different components to dissociate reversibly into its constituent components. The complex can be e.g. an antibodyantigen complex AbAg composed of antibody Ab and antigen Ag. $K_D$ is expressed in molar concentration [mol/L] and corresponds to the concentration of [Ab] at which half of the binding sites of [Ag] are occupied, in other words, the concentration of unbound [Ab] equals the concentration of the [AbAg] complex. The dissociation constant can be calculated according to the following formula:

$$\mathrm{K_D} = \frac{[\mathrm{Ab}] * [\mathrm{Ag}]}{[\mathrm{AbAg}]}$$

*[Ab]: concentration of antibody; [Ag]: concentration of antigen; [AbAg]: concentration of antibodyantigen complex*

In the context of the present specification, the terms *off-rate* (Koff;[1/s]) and *on-rate* (Kon; [L/s*mol]) are used in their meaning known in the art of chemistry and physics; they refer to a rate constant that measures the dissociation (Koff) or association (Kon) of an antibody with its target antigen. Koff and Kon can be experimentally determined using methods well established in the art. A method for determining the Koff and Kon of an antibody employs surface plasmon resonance. This is the principle behind biosensor systems such as the Biacore® or the ProteOn® system. They can also be used to determine the dissociation constant KD by using the following formula:

$$K_D = \frac{[K_{off}]}{[K_{on}]}$$

The natural upper limit for the on-rate $K_{on}$ is $10^9$ L/s*mol.

The term *aptamer* relates to an oligonucleotide or peptide molecule that binds to a specific target molecule. Aptamers can be created by selecting them from a large random sequence pool. Nucleic acid aptamers can be generated through repeated rounds of *in-vitro* selection or equivalently, SELEX (systematic evolution of ligands by exponential enrichment) to bind to molecular targets such as small molecules, proteins, or nucleic acids through non-covalent interactions. Aptamers offer molecular recognition properties that rival that of antibodies.

In the context of the present specification, the term *antibody* refers to whole antibodies including but not limited to immunoglobulin type G (IgG), type A (IgA), type D (IgD), type E (IgE), or type M (IgM), any antigen-binding fragment or single chains thereof and related or derived constructs. A whole antibody is a glycoprotein comprising at least two heavy (H) chains and two light (L) chains inter-connected by disulfide bonds. Each heavy chain is comprised of a heavy chain variable region ($V_H$) and a heavy chain constant region ($C_H$). The heavy chain constant region of IgG is comprised of three domains, $C_H1$, $C_H2$, and $C_H3$. Each light chain is comprised of a light chain variable region (abbreviated herein as $V_L$) and a light chain constant region ($C_L$). The light chain constant region is comprised of one domain, $C_L$. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component of the classical complement system. Similarly, the term encompasses a so-called nanobody or single domain antibody, an antibody fragment consisting of a single monomeric variable antibody domain.

The term *antibody-like molecule* in the context of the present specification refers to a molecule capable of specific binding to another molecule or target with high affinity / a Kd $\leq 10^{-8}$ mol/L. An antibody-like molecule binds to its target similar to the specific binding of an antibody. The term *antibody-like molecule* encompasses a repeat protein, such as a designed ankyrin repeat protein (Molecular Partners, Zürich), an engineered antibody mimetic protein exhibiting highly specific and high-affinity target protein binding (see US2012142611, US2016250341, US2016075767, and US2015368302, all of which are incorporated herein by reference). The term antibody-like molecule further encompasses, but is not limited to, a polypeptide derived from armadillo repeat proteins, a polypeptide derived from leucine-rich repeat proteins, and a polypeptide derived from tetratricopeptide repeat proteins. The term *antibody-like molecule* further encompasses a specifically binding polypeptide derived from a protein A domain, a fibronectin domain FN3, a consensus fibronectin domain, a lipocalin (see Skerra, Biochim. Biophys. Acta 2000, 1482(1-2):337-50), a polypeptide derived from a Zinc finger protein (see Kwan et al. Structure 2003, 11(7):803-813), an Src homology domain 2 (SH2) or Src homology domain 3 (SH3), a PDZ domain, a gamma-crystallin, ubiquitin, a cysteine knot polypeptide or a knottin, cystatin, Sac7d, a triple helix coiled-coil (also known as alphabodies), a Kunitz domain or a Kunitz-type protease inhibitor and a carbohydrate-binding module 32-2.

The term *protein A domains derived polypeptide* refers to a molecule that is a derivative of protein A and is capable of specifically binding the Fc region and the Fab region of immunoglobulins.

The term *armadillo repeat protein* refers to a polypeptide comprising at least one armadillo repeat, wherein an armadillo repeat is characterized by a pair of alpha-helices that form a hairpin structure.

The term "*extracellular vesicle*" (EV) refers to lipid bilayer-delimited particles that are naturally released from almost all types of cells. EVs range in diameter from near the size of the smallest unilamellar liposome (around 20-30 nanometers) to as large as 10 microns or more. The majority of EVs are smaller than 200 nm. EVs can be divided, according to their size and origin, into exosomes, microvesicles, and apoptotic bodies. EVs comprise proteins, nucleic acids, lipids, metabolites, and even organelles characteristic of their parent cell.

The term "exosome" refers to membrane-bound EVs that are produced in the endosomal compartment of most eukaryotic cells. Exosomes range in size from 30 to 150 nm.

## *Detailed Description of the Invention*

### *A method for isolating biomolecules, particularly for isolating extracellular vesicles*

A first aspect of the invention relates to a method for isolating a biomolecule from a liquid sample. The biomolecule may be present in the sample in solution, or may be present on the surface of a particle-like structure dispersed in the sample, as may be an extracellular vesicle, a cell, a virus, or virus-like-particle, or a biomolecule-coated solid nanoscale particle.

The method according to the invention proceeds in two steps, a binding step and release step, which for practical purposes in most embodiments will be separated by a washing step, in which unbound sample material is removed after the binding step.

In the binding step, the liquid sample comprising the biomolecule is brought into contact with a surface. The surface retains the biomolecule by one of two general mechanisms:

The surface may carry a chemical modification facilitating the retention of the biomolecule. This chemical modification may lead to electrostatic (ionic) bonding of biomolecules, van der Waals interaction or very specific antibody-like binding events.

Alternatively, the surface may allow electrical retention by applying a retention potential to said surface, facilitating retention of said biomolecule. The direction of the retention potential depends on the nature of the adsorbed entity and will be opposite to a release potential later used to release the entity. For example, negatively charged molecules will be adsorbed by a positive retention potential, and released by a negative release potential (release voltage).

In certain particular embodiments, the surface comprises a ligand capable of specifically and non-covalently binding to the biomolecule, exemplified by but not limited to an antibody, the ligand being attached to the surface. The surface is or covers an electrically conductive material so that it can be used as an electrode and the specific ligand-biomolecule interaction can be disturbed to the point of release by applying a release voltage to the surface in the release step.

In the binding step, the biomolecule is thus bound to the surface by means of the previously bound ligand. In embodiments where the biomolecule is attached to an extracellular vesicle, cell, virus, or other particle, this results in it being bound to the surface.

In a subsequent washing step, the liquid sample is removed. Optionally, the surface is washed to remove remaining impurities that are retained on the surface initially after the removal of the sample.

A collection buffer or solution is provided to cover the surface having the biomolecule attached thereto.

In a release step, a release voltage or electrical potential is applied to the surface, thereby releasing the biomolecule and, where applicable, the extracellular vesicle, eukaryotic or prokaryotic cell, virus, or particle from the surface. The inventors have applied a DC release voltage in their examples, but an AC release voltage might also be employed.

Subsequently, the biomolecule is collected in the collection solution/buffer.

According to the invention, the surface is an electrically conductive micro- or nanostructured material. Only such material allows the necessary packing densities to achieve meaningful amounts of ligand to be attached, and meaningful amounts of biomolecule or particles to be recovered.

In certain embodiments, the method for isolating extracellular vesicles facilitates loading the EV with cargo molecules that do not diffuse into the EV without disrupting the EV membrane. This method further to the binding, optional washing, and release step comprises a loading step conducted subsequent to the binding step, and prior to the release step, wherein the surface is contacted with a loading solution comprising a cargo molecule to be transferred into the extracellular vesicle.

A loading voltage having a polarity opposite to a polarity of the release voltage is applied to the surface, thereby allowing entry of the cargo molecule into the extracellular vesicle.

*A method for loading biological vesicles, particularly EV*

Another aspect of the invention relates to a method for loading a biological vesicle as can be an extracellular vesicle, a eukaryotic or a prokaryotic cell, with a cargo molecule. A particular application is the loading of extracellular vesicles, more particularly exosomes. The method according to this aspect of the invention comprises the steps of contacting a surface provided by an electrically conductive micro- or nanostructured material with an aqueous medium. The aqueous medium comprises an extracellular vesicle and a cargo molecule to be transferred into the extracellular vesicle. A loading voltage is applied to the surface, thereby allowing entry of the cargo molecule into the extracellular vesicle.

In certain embodiments, the cargo molecule is selected from the group comprising a pharmaceutical drug, a protein, a nucleic acid, a dye molecule.

In certain particular embodiments, the cargo molecule is selected from the group comprising a small molecule pharmaceutical drug, and a nucleic acid oligomer (single or double stranded) having a length of 10 to 150 nucleotides. Nucleic acid oligomers encompass siRNA, shRNA, antisense oligonucleotides including such that comprise thioate bonds, Locked Nucleic Acid moieties or both.

The examples contain an embodiment wherein electroporation occurs in an aqueous solution so that the total resistance of the cell (the resistance between the contact of the working electrode and the counter electrode) is higher than 600 Ω (ohm) i.e. at low ionic strength, when the distance between the electrodes is about 200 μm. The resistance during the experiment shown in the example was 1 M Ω.

*The biomolecule and particles attached thereto*

In principle, the method can be employed to generate a reversible, instant-on-off affinity column where any molecular structure recognized and specifically bound by an antibody or similar interaction can be absorbed and then released by applying an electrical voltage.

The method and device presented here were specifically developed to facilitate the collection of extracellular vesicles, a biological entity that has recently become the focus of intense scientific interest. Exosomes, microvesicles, and apoptotic bodies have all been recognized for their diagnostic significance and therapeutic potential.

In certain embodiments, the biomolecule isolated by the method according to the invention is presented on a biological particle and thus, the biological particle is isolated.

In certain particular embodiments, the biomolecule isolated by the method according to the invention is presented on an extracellular vesicle. In certain embodiments, the biomolecule isolated by the method according to the invention is presented on a eukaryotic or prokaryotic cell or a virus.

In certain embodiments, the biomolecule is a soluble biomolecule not associated with a cell, to a vesicle, or particle.

*The conductive micro- or nanostructured material*

The surface to which the biomolecule or biomolecule-attached vesicle, particle, or cell is bound in the binding step is an electrically conductive material characterized by the structure of micro- or nanoscale dimension, in other words, an electrically conductive micro- or nanostructured material.

In the context of the present specification, the term "carbon microfiber mesh" refers to a carbon electrode material characterized by at least one nanoscale or microscale dimension.

In the context of the present specification, the term "microscale dimension" signifies that at least one dimension of the electrode is lower than 1 mm, or ranges from 0.1 micrometers to 900 micrometers. In the context of the present specification, the term "nanoscale dimension" signifies that at least one dimension of the electrode is lower than 1 micrometer, or ranges from 0.1 nanometers to 900 nanometers. The nanoscale or microscale dimension depends on the shape of the electrode. If an electrode is generally shaped like a cylinder, then at least one dimension can be one or both of a length and a diameter of the electrode. If the electrode is generally shaped as rectangular, then at least one dimension can be one or more of a length and a width of the electrode. Electrodes characterized by one or more microscale or nanoscale dimensions are also referred to herein, and are generally known in the art, as "microelectrodes".

In the context of the present specification, the term carbon fiber (CF) electrode refers to an electrode that comprises, particularly consists of, elementary carbon (e.g., graphite) shaped as a fibrous structure (e.g., a filament). Generally, but not necessarily, a CF electrode is characterized by a microscale or even nanoscale diameter or thickness (width), typically of, but not limited to, a range from 1 to 500 micrometers, or from 5 to 200 micrometers, or 5 to 100 micrometers, or 5 to 50 micrometers or 5 to 20 micrometers. Generally, but not necessarily, a CF electrode is characterized by a length (height) from about 100 micrometers to about 50 mm, or from about 100 micrometers to about 1 mm, or from about 100 micrometers to about 800 micrometers, including any intermediate values and subranges there between. A CF electrode featuring at least one dimension in the microscale or nanoscale range is also termed a "CF microelectrode".

In certain embodiments, the conductive micro- or nanostructured material is selected from carbon microfibers, metal microfibers, nanowires, nanopillars, micropillars, glassy carbon, screen-printed carbon, carbon films, carbon fibers, carbon paste, and carbon nanotubes.

A *nanowire* is a nanostructure with a diameter of the order of a nanometre. Nanowires can be defined as structures that have a thickness or diameter constrained to tens of nanometres or less and an unconstrained length. At these scales, quantum mechanical effects are important - which coined the term "quantum wires". Many different types of nanowires exist, including superconducting (e.g. YBCO[1]), metallic (e.g. Ni, Pt, Au, Ag), semiconducting (e.g. silicon nanowires (SiNWs), InP, GaN), and insulating (e.g. SiO2, TiO2).

*Nanopillars* are an emerging technology within the field of nanostructures. Nanopillars are pillar-shaped nanostructures approximately 10 nanometers in diameter that can be grouped together in a lattice like arrays (Fan et al., DOI: 10.1021/n11010788 | Nano Lett. 2010, 10, 3823-3827).

In certain embodiments, the conductive micro- or nanostructured material is a carbon fiber (CF) microelectrode. In certain particular embodiments, the conductive micro- or nanostructured material is a carbon microfiber mesh.

In certain embodiments, the CF microelectrode further comprises mechanical support enveloping or surrounding at least a portion of the electrode, leaving a protruding tip of the unsupported, exposed portion of the electrode. In certain particular embodiments, such exposed portion ranges from 10 to 100 micrometers in size.

The CF microelectrode can be a single-barrel or a multi-barrel electrode.

In certain embodiments, a CF microelectrode is a gas-permeable electrode.

Two methods are generally used in the art to characterize air permeability:

1. Through-plane air permeability at 200 Pa pressure drop (DIN EN ISO 9237). In practice, this measurement is performed at 100-1000 L / $m^2 \cdot s$.

2. Through-plane Air Permeability acc. to Gurley (ISO 5636-5). This method measures the amount of time required for a certain volume of air to pass through a test specimen.

The term "gas-permeable" in the context employed here refers to a property of the electrode characterized by air permeability through-plane higher than 100 to 1000 L/ $m^2 \cdot s$ when measured according to the standard assay defined by ISO 9237 through 0.25 mm.

Advantages of using a gas-permeable electrode include

- its better integration with the non-electrically conductive polymer matrix (in particular embodiments: PDMS) matrix. The polymer is capable of diffusing and easily connecting to the electrode;
- the provision of a high surface area for interaction with the sample due to 3D permeable matrix.

It is understood that the term "gas permeable" characterizes the electrode material prior to any embedding step that submerges the electrode material partially into a polymer matrix. The matrix material, particularly PDMS, has a very low permeability to air. The matrix material constitutes the seal/walls of the fluidic channel.

The electrode material used in the invention does not have to be gas permeable but must contain a large surface area and a three-dimensional structure.

*Thickness at 1 MPa 10-10000 $\mu$m (particularly: 100-300 $\mu$m) of the conductive micro- or nanostructured material:*

In certain embodiments, the conductive micro- or nanostructured material is a CF microelectrode characterized by a mass per unit area (DIN EN ISO 29073-1) from 1 to 1000 g/$m^2$. In certain particular embodiments, the conductive micro- or nanostructured material is a CF microelectrode characterized by an area weight of 30-200 g/$m^2$.

In certain embodiments, the conductive micro- or nanostructured material is a CF microelectrode characterized by a through-plane electrical resistance at 1 MPa of 0.1 to 100 mΩ·cm. In certain particular embodiments, the conductive micro- or nanostructured material is a CF microelectrode characterized by a through-plane electrical resistance at 1 MPa of 2-20 $m\Omega \cdot cm^2$.

In certain embodiments, the conductive micro- or nanostructured material is a CF microelectrode characterized by an in-plane electrical resistance from 0.05 to 20 Ω. In certain embodiments, the conductive micro- or nanostructured material is a CF microelectrode characterized by an in-plane electrical resistance from 0.5 to 2 Ω. These values were provided as material characteristics by the maker of the electrode material, Fuel Cell Store, College Station, TX, USA (https://www.fuelcellstore.com).

In certain embodiments, the conductive micro- or nanostructured material is a CF microelectrode characterized by a through-plane air permeability at 200 Pa pressure drop (DIN EN ISO 9237) from 100 to 1000 L/$m^2 \cdot s$.

In certain embodiments, the conductive micro- or nanostructured material is a CF microelectrode characterized by an in-plane Air Permeability at 1 MPa from 0.2 to 5 $\mu m^2$.

In certain embodiments, the CF microelectrode is a carbon fabric electrode or a carbon paper electrode, in particular, a gas-permeable carbon fabric or carbon paper electrode. Carbon fabric electrodes can be made of woven or non-woven carbon filaments or bundles of filaments.

Exemplary commercially available gas-permeable carbon fabric microelectrodes that are usable in the context of the present embodiments include, but are not limited to, plain carbon cloth such as, for example, electrodes marketed as ELAT—Hydrophilic Plain Cloth®, 1071 HCB plain carbon cloth, Panex 30®.

In certain particular embodiments described herein, the CF microelectrode is a carbon paper electrode.

Exemplary commercially available gas-permeable carbon paper microelectrodes that are usable in the context of the present embodiments include, but are not limited to, electrodes marketed by Freudenberg FCCT, such as Freudenberg H23, electrodes of the Spectracarb™ family, Sigracet 39 AA, electrodes marketed under the trade name AvCarb® (e.g., AvCarb P75), and similar gas-permeable carbon paper electrodes.

Any commercially available CF microelectrode can serve as a raw material for providing a CF microelectrode according to the present embodiments, upon generating on at least a part of its surface a functional moiety as described herein.

In certain embodiments, the CF microelectrode is characterized by a surface area of at least 10-50 $cm^2$ per geometrical $cm^2$.

In certain embodiments, the CF microelectrode is electrically connectable to other parts of a potentiostat/electrical system via electrically conducting wires or needles, for example, conducting metal steel needles or metal foils exemplified by, but not limited to, nickel, aluminium, or copper foils.

In certain embodiments, the conductive micro- or nanostructured material, particularly the CF microelectrode, is embedded in a polymer matrix, and an exposed portion of the conductive micro- or nanostructured material is exposed to said liquid sample and or said loading solution. In certain particular embodiments, the exposed portion protrudes 0.5 μm to 1 mm into the liquid sample. The inventors contemplate a broader range that in certain embodiments might still be capable of providing the advantages of the invention, such as from 0.01 μm to 10 mm.

*The surface*

The CF microelectrode of the present invention in any of its aspects and embodiments comprises an attachment layer. In certain embodiments, this attachment layer can be a chemically/electrostatically charged layer enabling interaction with a vesicle on the basis of ionic charge. In certain embodiments, this can be a hydrophobic/hydrophilic layer, interacting with a vesicle on the basis of non-covalent forces such as van-der-Waals interaction.

In certain embodiments, the surface is functionalized with short (C2-C20) alkyl moieties comprising positively charged (cationic) or negatively charged (anionic) groups. In certain embodiments, such cationic moieties are selected from amino, C1-C4 alkylamino and carbamoyl. In certain embodiments, such anionic moieties are selected from carboxyl, phosphate, phosphonate, sulfate.

If a very high surface area is functionalized, a situation can be created where the surface can form many weak bonds which together can capture cells or vesicles. Moieties like hydroxyl, C1-C20 alkyl, carbonyl, sulfhydryl groups can be employed alternatively to functionalize the surface. See: Manneli et al., Langmuir 2016, 32, 48, 12632-12640.

Many chemical methodologies are known for functionalizing carbon or metal surfaces. See Steeno et al., Chem. Mater. 2020, 32, 12, 5246-5255; Zhang et al., J. Am. Chem. Soc. 2019, 141, 20, 8277-8288; Nayak et al. in Carbon-Containing Polymer Composites pp 65-98, Springer 2018; Khan and Nishina (Nanoscale 2021, 2021,13, 36-50).

*The surface comprises* a *ligand*

In certain particular embodiments, the surface is covalently attached to a ligand, in otherwords, a functional moiety capable of engaging and retaining the biomolecule in at least a portion of its surface (e.g., an exposed portion of the electrode's surface). It is understood that the expression "a ligand" molecularly speaking refers to a plurality of ligands or functional moieties capable of retaining a relevant number of biomolecules.

In certain embodiments, the functional moiety is covalently attached to the surface.

In certain embodiments, when the ligand is attached to the surface via a hydrocarbon chain ("crosslinker"), the hydrocarbon chain can be of any length. For example, the linker chain can be of 1 to $10^6$, or of 1 to $10^3$, or from 1 to 100, or from 1 to 50, or from 1 to 20, or from 1 to 10, atoms in length, including any intermediate values and subranges therebetween.

The carbon backbone of the crosslinker chain can be hydrophobic or hydrophilic. For example, poly(ethyleneglycol) could serve as the crosslinker, rather than a straight alkyl chain. The length of the crosslinker should not affect the activity of the ligand, particularly the antibody. The density of the crosslinker could vary, and could affect the hydrophobicity of the environment.

In certain embodiments, an average density of the ligands on the CF microelectrode surface is at least $10^{10}$ ligand moieties per $cm^2$, or at least $10^{11}$ ligand moieties per $cm^2$, and can be, for example, from about $10^{10}$ to about $10^{13}$, or from about $10^{11}$ to about $10^{13}$, ligand moieties per $cm^2$ of the electrode's surface.

Antibody molecules can be attached to various substrates by covalent, hydrophobic, electrostatic, or van der Waals forces. Antibodies are soft proteins that adsorb on various substrates by spreading. The antibody immobilization density has been observed to be higher for adsorption than for covalent binding. Antibodies have also been bound to amino-silane (like APDMES and APTES) functionalized substrates by passive adsorption that involves strong ionic interactions. IgG has a molecular weight of 156 kDa and dimensions of $14.5 \times 8.5 \times 4$ nm while the area covered by an IgG molecule with side-on orientation is minimal and has Ab immobilization density of ~2.6 $mg/m^2$. The structure of Ab is unaffected by the incubation of IgG with the silanized substrate.

The device and method according to the invention can be used in many applications, including without limitation, chemical applications, genetic applications, biochemical applications, pharmaceutical applications, biomedical applications, medical applications, radiological applications and environmental applications.

For medical applications, the system and method of the present embodiments is suitable for monitoring the presence, and more preferably level, of biological entities in a biological liquid, such as a physiological solution.

For environmental applications, the system and method of the present embodiments is suitable for monitoring presence, and more preferably level, biological entities indicative of the presence or level of hazardous materials in a liquid, such as, but not limited to, water pollutants, chemical agents, biological organisms or radiological conditions in water.

The liquid can be a liquid that comprise a blood product, either whole blood or blood component. For example, the liquid can be a blood sample. The liquid can comprise other body liquids, including, without limitation, saliva, cerebrospinal fluid, urine, and the like. The liquid can be a buffer or a solution, such as but not limited to, nucleic acid solutions, protein solutions, peptide solutions, antibody solutions, and the like. Also contemplated are liquids containing one or more biological and chemical reagents such as, but not limited to, oxidizing agents, reducing agents, enzymes, receptor ligands, extracellular components, metabolites, fatty acids, steroids, and the like. A representative list of liquids in which the system and method of the present embodiments can capture a marker/analyte include, without limitation, water, saltwater, urine, blood, sperm, saliva, mucous, catamenial fluid, lymphatic fluid, cerebrospinal fluid, vaginal exudate, pus, gastrointestinal fluid, perspiration, tears, vitreous body.

The interaction between biomolecule and ligand can be characterized by a $K_D$ which is equal or less than $10^{-5}$ M, particularly by a $K_D$ which is equal or less than $10^{-6}$ M, more particularly by a $K_D$ which is equal or less than $10^{-7}$ M, even more particularly by a $K_D$ which is equal or less than $10^{-8}$ M or a $K_D$ which is equal or less than $10^{-9}$ M or a $K_D$ which is equal or less than $10^{-10}$ M. The biomolecule comprised in the sample, or the vesicle attached thereto, binds selectively to the ligand.

After the attachment of the biomolecule to the electrode surface, the electrode is washed with a washing buffer. This results in the desorption of background components from the surface of the electrode. Since the interactions between the biomolecule and the ligand are stronger than the interaction of the background components with the surface of the electrode, the background components leave the electrode's surface much faster than the biomolecule. The biomolecule's desorption kinetics from the electrode can be detected after all or most of the background components are washed out.

While the embodiments below are described with a particular emphasis to biomolecules in a biological liquid, it is to be understood that other types of molecules and other types liquid are also contemplated.

The system of the present embodiments optionally provides direct analysis of samples on a single device. The system of the present embodiments can selectively detect specific low abundant biomarker biomolecules while removing unwanted components (salts, non-specifically bound non-target bio-molecules, proteins, cells, etc.). In certain embodiments, the analysis is performed without performing at least one of, or more particularly without any of centrifugation, desalting, and affinity columns, since such operations are known to be time-consuming. In some embodiments of the present invention, the analysis process is performed in less than 180 minutes or less than 15 minutes or less than 10 minutes or less than 5 minutes.

In certain embodiments, the ligand is selected from an antibody, an antibody-like molecule, an aptamer, and a DARPin. Any biological ligand showing high specificity for an antigen-like structure mediated by non-covalent binding can be attached to the surface. In principle, if the biomolecule to be collected and isolated is an antibody or similar molecule itself, its binding partner -the antigen- might be the one attached to the surface. In cases, for example, where the aim is to remove certain vesicle-like structures bearing specific B cell receptors or antibodies from the circulation or isolating such structures, the antigen to such antibodies may be attached to the surface.

In certain embodiments, the antibody is a gamma immunoglobulin, a gamma immunoglobulin Fab fragment, a diabody, or a nanobody.

In certain embodiments, the biomolecule is a membrane protein, particularly a membrane protein embedded in the membrane of an extracellular vesicle.

In certain embodiments, an extracellular vesicle is isolated.

In certain embodiments, the surface is coated with a material providing cationic moieties, particularly wherein the surface is coated with a material providing alkylamine moieties, more particularly wherein the surface is coated with an aminoalkylsilane, even more particularly wherein the surface is coated with 3-aminopropyl triethoxysilane (APTES), 3-aminopropyl methyl diethoxysilane (APMDES) or 3-aminopropyl dimethyl ethoxysilane (APDMES).

In such embodiments, the material providing cationic moieties covers the electrode with a monolayer or multilayer. It may also cover the surface of the matrix, such as exemplified by PDMS in the examples.

In order to conjugate ligands (affinity moieties) to the surface, the electrode can be modified either covalently or electrostatically. The person skilled in the art of surface modification chemistry is able to readily chose chemical methods by which ligands can be immobilized on the surface electrode, including but not limited to aminoalkyl silanes. Groups available for conjugation include carboxylic acids, carboxylic acid esters, carboxylic acid amines, azides, carbonyl/aldehyde groups, hydroxyls, sulfonyl, sulfonyl chlorides, epoxide, or an activated derivative thereof (for example, an n-hydroxysuccinimide (NHS) ester moiety).

The examples provided herein (see Ex. 3b) illustrate how an antibody can be bound covalently to the aminated surface. The skilled artisan understands that this is but one of many methods by which a ligand can be attached to a surface in preparing the method and device of the invention.

Other methods of covalent Ab immobilization are known to the skilled artisan and reviewed, inter alia, in Dennler et al., Antibodies 2015, 4(3), 197-224.

Electrostatic adsorption can be performed with polyaniline, polylysine, and similar agents available to the skilled artisan.

In certain embodiments, the ligand is capable of specifically binding to an exosome-specific marker, particularly a marker selected from CD9, CD63, CD81, CD82, and CD86.

In certain embodiments, the ligand is capable of specifically binding to non-specific surface protein, particularly a protein selected from CD16, CD18, CD14, CD13, MCT-1, Na/K ATPase, Cd11b/Mac-1, MHC I and II, IL-1β, Flotillin-1, an integrin, an annexin, or a lipid-raft sphingolipid, cholesterol, or a ceramide.

*Potentials used in the methods of the invention*

In certain embodiments, the release voltage ranges from -3 V to 3 V, particularly for negatively charged particles or biomolecules in the release buffer from 0 V to -3 V, or for positively charged particles or biomolecules in the release buffer from 0 V to 3 V.

In certain embodiments of the method disclosed herein, the inventors use -1.6 V to release the extracellular vesicles (negatively charged entities).

For negatively charged entities that are released by applying a negative potential, using voltages that are lower than -2 V risks destruction of their chemical bonds and irreversible change. In certain embodiments, the potential applied ranges from -0.1 V to -2.0 V. In certain particular embodiments, the potential applied ranges from -0.2 V to -1.8 V.

For positively charged entities, that are released in positive potentials, using voltages that are higher than +2 V risks destruction of their chemical bonds and irreversible change. In certain embodiments, the potential applied ranges from +0.1 V to +2.0 V. In certain particular embodiments, the potential applied ranges from +0.2 V to +1.8 V.

When applying water electrolysis potentials, $H^+$ cations will be consumed/reduced at the cathode and $OH^-$ anions will be consumed/oxidized at the anode. The environment near the cathode is basic while the environment near the anode is acidic. These local pH changes can contribute to the release kinetics of the biological entities from the electrode's surface by a conformational change of the antibody or/and antigen.

The potential could theoretically have much higher values, but practically the range from -3 V to +3 V has proven the range providing the best benefit, as decomposition issues affecting the electrode create impurities in the process at higher voltages.

In certain embodiments, the loading voltage ranges from -350 V to -50 V or from +350 V to +50 V, particularly wherein the loading voltage ranges from -300 V to -100 V or from +300 V to +100 V.

In certain particular embodiments, the loading voltage is applied as a loading voltage burst for 0.5 ms to 5 ms, particularly for about 1 ms,

In certain more particular embodiments, 2 to 20 loading bursts are applied.

In certain embodiments, the retention voltage ranges from +3 V to -3 V, particularly from +2 V to -2 V, even more particularly from +1.5 V to -1.5 V, yet even more particularly from +1 V to - 1 V.

In general, the retention voltage is of the same order of magnitude as the release voltage but with the opposite potential sign, i.e. negative potential for retention, when a positive potential is used for release, and vice versa.

*The device for practicing the method of the invention can be a flow chamber*

In certain particular embodiments, the surface is comprised in a flow chamber, and the sample is passed through the flow chamber without an electrical potential being applied during the binding step and the washing step.

In certain embodiments, the surface forms a working electrode constituting a longitudinal wall of the flow chamber, and a counter electrode is present opposite the working electrode.

The counter electrode does not need to be made of the same material as the working electrode (e.g., carbon mesh/ CF), but can be any other electrode with a high surface area. In certain embodiments, the counter electrode is coated with ligand (e.g., antibodies). If the counter electrode is covered with ligand, and thus employed for biomolecule retention, the release voltage applied to the poles of the electrochemical cell during the release process can be switched during the release step, in order to liberate biomolecules from the counter electrode as well.

In certain embodiments, the working electrode and the counter electrode are separated by a distance ranging from 1 μm to 100 mm. In certain particular embodiments, the working electrode and the counter electrode are separated by a distance ranging from 0.2 mm to 0.6 mm, which characterizes the range of separations for which the inventors performed experimental valuations.

This distance dictates the ratio of active surface/volume of sample. Hence, the higher the distance separating the electrodes, the lower the fraction of the solution that is processed at each cycle, and the lower is the concentration of purified analyte that is released to the collection buffer. Furthermore, the distance between the electrodes also dictates the type of sample that can be injected into the device: solutions with higher resistance to flow are defined by higher viscosity, and should be injected into a device with a higher distance between the electrodes.

*A device for practicing the method of the invention*

A second aspect of the invention relates to a device, particularly a flow cell, for isolating a biomolecule. The device comprises a chamber configured for receiving a liquid sample.

The device according to the invention is characterized in that a first surface of the chamber is formed by a high-surface, electrochemically active electrically conductive material embedded in a non-electrically conductive polymer matrix. The electrochemically active material is linked to a ligand capable of specifically binding to a biomolecule, as already discussed above in relation to the first aspect of the invention in any of its embodiments. This first surface forms a working electrode.

The device according to the invention further comprises a counter electrode configured to contact the liquid sample.

A first and second electrically conductive connection connectable to a voltage source are connected to the working and counter electrode, respectively. Importantly, the contacts can be switched, to form a counter electrode from (2) and a working electrode from (1). This is used at the release stage after first release, in order to release also bound EVs from the counter electrode.

In certain embodiments, a second surface of said chamber is formed by an electrically conductive material forming a counter electrode.

In certain embodiments, a third surface of said chamber is formed by an electrically conductive material forming a reference electrode.

In certain particular embodiments, the chamber forms a flow cell. The flow cell comprises an inlet port and an outlet port separated by a cell volume, and the working electrode is positioned opposite the counter electrode between the inlet port and the outlet port.

For preconcentration applications, the ratio between the inner volume of the channel and the electrodes' geometrical surface is critical. The lower the height of the channel, the better the preconcentration, and the longer it takes to process large samples. In practice, the length and width can vary from 1 mm to 1000 mm. The channel height can vary between 0.001 mm to 100 mm.

In certain embodiments, the high-surface, electrochemically active material is embedded in a polymer matrix, and an exposed portion of the conductive micro- or nanostructured material is exposed to said liquid sample and or said loading solution. In particular embodiments, the electrochemically active material embedded in a polymer matrix is a CF microelectrode.

In certain particular embodiments, the exposed portion protrudes 0.5 μm to 1 mm into the liquid sample.

In certain embodiments, the conductive micro- or nanostructured material, particularly the CF microelectrode, is characterized by any of the following parameters:

a. Density less than 1.5 g/cm$^3$, more particular: 1.1-0.1 g/cm$^3$,

b. Area density: 200-30 g/m$^2$,

c. In-plane electrical resistance (van der Pauw method): 1.5-0.015 Ω·mm.

d. Resistivity of $10^{-3}$ -$10^{-6}$ Q·m at 20 °C;

e. Suitable to perform electrochemical analysis (cyclic voltammetry) between -0.8 V to +0.8 V (versus reference electrode).In certain embodiments, the non-electrically conductive polymer matrix is selected from an elastomer, thermoplastic elastomer and a thermosetting polymer.

In certain embodiments, the non-electrically conductive polymer matrix comprises, particularly consists of, a material selected from the group comprising polystyrene, polycarbonate, polyacrylate, poly-methyl methacrylate, poly-ethylene glycol diacrylate, polyethylene, polyurethane, acrylonitrile butadiene styrene, nylon (polyamides), polylactic acid, polybenzimidazole, polyether sulfone, polyoxymethylene, polyether ether ketone, polyetherimide, polyphenylene oxide, polyphenylene sulfide, polypropylene, polyvinyl chloride, polyvinylidene fluoride, polytetrafluoroethylene (Teflon).

In certain embodiments, the non-electrically conductive polymer matrix comprises, particularly consists of, a polyester resin, a polyurethane, vulcanized rubber, polyoxybenzylmethylenglycolanhydride (bakelite), urea-formaldehyde (melamine) resin, diallyl-phthalate (DAP), an epoxy resin, epoxy novolac resins, a benzoxazine, a polyimide, a bismaleimide, a cyanate ester, a furan resin, a silicone resin, Thiolyte®, a vinyl ester.

The optimal performance was achieved with 58 mm × 8 mm × 0.2 mm, which has an inner volume of 93 μL (without the tubing).

The present invention provides a novel purification device with high throughput capabilities. The device shown in the examples is composed of a 3-dimensional (3D) micro-carbon fibers electrode bearing a high surface area embedded in a polydimethylsiloxane (PDMS)-based fluidic channel. Surface conjugation with affinity agents such as antibodies and their fragments, nanobodies, designed ankyrin repeat proteins (DARPins), lectins, or aptamers against EVs surface proteins allows specific and selective separation. The controlled release into a selected buffer is driven by electrical voltage. No further processing steps are required. The design of the fluidic channel and electrodes does not allow blockage or clogging. The entire purification process occurs within a time frame of 10 min. Furthermore, this device can be optimized for automatization and perform as a plug-and-play system.

The device according to the invention bears the following advantages over existing isolation techniques: (1) easy-to-use, (2) rapid, (3) high- throughput, (4) high-yield, (5) highly reproducible fabrication procedure, (6) inexpensive, and (7) able to process clinical samples of both small and large volumes with high purity. Isolation of large amounts of EVs straight from biological fluids would enable thorough investigation into their therapeutic activity and may allow to manipulate them for drug delivery. The device is fast, efficient, cost-effective, and suitable for use with a broad range of biological samples and volumes. This device could potentially become a standard method for exosome isolation, purification, and even modification. allowing comparing data between laboratories.

The present invention, in some embodiments thereof, relates to purification, capturing, and release of EVs and, more particularly, but not exclusively, to novel fluidic electrochemical systems and methods employing the same, which are usable in purification modification and detection of EVs such as exosomes, EVs, proteins, protein aggregates, viruses, bacteria, platelets, microvesicles, apoptotic bodies.

Wherever alternatives for single separable features such as, for example, a ligand or surface or isolated structure are laid out herein as "embodiments", it is to be understood that such alternatives may be combined freely to form discrete embodiments of the invention disclosed herein.

The invention further encompasses the following items:

1. A method for capturing a biomolecule, said method comprising

a. in a binding step, contacting a liquid sample comprising said biomolecule with a surface, wherein

i. said surface is electrically conductive,

ii. and

- said surface carries a chemical modification facilitating retention of said biomolecule, or

- an electrical retention potential is applied to said surface, facilitating retention of said biomolecule;

b. in a washing step, removing said liquid sample;

c. in a release step, applying a release voltage to said surface,

d. collecting the biomolecule;

characterized in that
said surface is an electrically conductive micro- or nanostructured material.

2. The method according to item 1, wherein the surface comprises a ligand capable of specifically binding to said biomolecule.

3. The method according to item 1 or 2, wherein the biomolecule is presented on a biological particle, particularly an extracellular vesicle, a eukaryotic or prokaryotic cell, or a virus, more particularly wherein the biomolecule is presented on an extracellular vesicle, even more particularly wherein the biomolecule is presented on an exosome.

4. The method according to item 1 or 2, wherein the biomolecule is a soluble biomolecule not associated with a cell or particle.

5. The method according to item 3, wherein the biomolecule is presented on a biological vesicle, particularly an extracellular vesicle, more particularly wherein the biomolecule is presented on an exosome, and wherein

a. in a loading step conducted subsequent to the binding step, and prior to the release step, the surface is contacted with a loading solution comprising a cargo molecule,

b. a loading voltage having a polarity opposite to a polarity of the release voltage is applied to the surface.

6. A method for loading a biological vesicle, particularly an extracellular vesicle, with a cargo molecule, comprising the steps:

a. providing an aqueous medium comprising a biological vesicle, particularly an extracellular vesicle, wherein the aqueous medium is in contact with a surface that is electrically conductive, wherein the aqueous medium is a loading solution comprises a cargo molecule;

b. applying a loading voltage to the surface;

characterized in that
said surface is an electrically conductive micro- or nanostructured material.

7. The method according to item 5 or 6, wherein the cargo molecule is selected from the group comprising a pharmaceutical drug, a protein, a nucleic acid, a dye molecule,
particularly wherein the cargo molecule is selected from the group comprising a small molecule pharmaceutical drug, and a nucleic acid oligomer having a length of 10 to 150 nucleotides.

8. The method according to any one of the preceding items, wherein the conductive micro- or nanostructured material is selected from the group comprising carbon microfibers, metal microfibers, nanowires, nanopillars, micropillars, glassy carbon, screen-printed carbon, carbon films, carbon fibers, carbon paste, carbon nanotubes.

9. The method according to any one of the preceding items, wherein the conductive micro- or nanostructured material is a carbon fibre (CF) microelectrode, particularly a carbon microfiber mesh.

10. The method according to any one of the preceding items, wherein the conductive micro- or nanostructured material, particularly the CF microelectrode, is embedded in a polymer matrix, and an exposed portion of the conductive micro- or nanostructured material is exposed to said liquid sample and or said loading solution,
particularly wherein the exposed portion protrudes 0.5 $\mu$m to 1 mm into the liquid sample.

11. The method according to any one of the preceding items 1 to 10, wherein the conductive micro- or nanostructured material, particularly the CF microelectrode, is characterized by any of the following parameters:

a. Density less than 1.5 g/cm$^3$, more particular: 1.1 - 0.1 g/cm$^3$,
b. Area density: 200 - 30 g/m$^2$,
c. In-plane electrical resistance (van der Pauw method): 1.5 - 0.015 $\Omega$·mm.
d. Resistivity of $10^{-3}$ - $10^{-6}$ Q·m at 20 °C;
e. Suitable to perform electrochemical analysis (cyclic voltammetry) between -0.8 to +0.8 V (vs reference electrode).

12. The method according to any one of the preceding items 2 to 11, wherein the ligand is covalently attached to the surface.

13. The method according to any one of the preceding items 2 to 12, wherein the ligand is selected from the group comprising an antibody, an antibody-like-molecule, an aptamer, a DARPin.

14. The method according to item 13**Fehler! Verweisquelle konnte nicht gefunden werden.,** wherein the antibody is selected from the group comprising a gamma immunoglobulin, a gamma immunoglobulin Fab fragment, a diabody, a nanobody.

15. The method according to any one of the preceding items, wherein the biomolecule is a membrane protein.

16. The method according to any one of the preceding items, wherein an extracellular vesicle is isolated.

17. The method according to any one of the preceding items, wherein the surface is coated with a material providing cationic moieties, particularly wherein the surface is coated with a material providing alkylamine moieties, more particularly wherein the surface is coated with an aminoalkylsilane, even more particularly wherein the surface is coated with 3-aminopropyl triethoxysilane (APTES), 3-aminopropyl methyl diethoxysilane (APMDES) or 3-aminopropyl dimethyl ethoxysilane (APDMES).

18. The method according to any one of the preceding items, wherein the ligand is capable of specifically binding to an exosome-specific marker, particularly a marker selected from CD9, CD63, CD81, CD82, and CD86.

19. The method according to any one of the preceding items, wherein the ligand is capable of specifically binding to non-specific surface protein, particularly a protein selected from CD16, CD18, CD14, CD13, MCT-1, Na/KATPase, Cd11b/Mac-1, MHC I and II, IL-1β, Flotillin-1, an integrin, an annexin, or a lipid-raft sphingolipid, cholesterol, or a ceramide.

20. The method according to any one of the preceding items, wherein the release voltage ranges from -3 V to 3 V, particularly from 0 V to -3 V, or from 0 V to 3 V.

21. The method according to any one of the preceding items 4 to 20, wherein the loading voltage ranges from -350 V to -50 V or from +350V to +50V, particularly wherein the loading voltage ranges from -300 V to -100 V or from +300 V to +100 V.

22. The method according to any one of the preceding items 4 to 21, wherein the loading voltage is applied as a loading voltage burst for 0.5 ms to 5 ms, particularly for about 1 ms,

23. The method according to item22, wherein 2 to 20 loading bursts are applied.

24. The method according to any one of the preceding items 4 to 23, wherein the retention voltage ranges from +3 V to -3 V, particularly from +2 V to -2 V, even more particularly from +1.5 V to -1.5 V, yet even more particularly from +1 V to -1 V.

25. The method according to any one of the preceding items, wherein the surface is comprised in a flow chamber, and the sample is passed through the flow chamber.

26. The method according to item 25, wherein the surface forms a working electrode constituting a longitudinal wall of the flow chamber, and a counter electrode is present opposite the working electrode.

27. The method according to item 26, wherein the working electrode and the counter electrode are separated by a distance ranging from 1 μm to 100 mm.

28. A device for isolating of a biomolecule, or for isolating and/or loading of an extracellular vesicle, said device comprising a chamber configured for receiving a liquid sample, wherein

- a first surface of said chamber is formed by a high-surface, electrochemically active material embedded in a non-electrically conductive polymer matrix, said first surface forming a working electrode (2);
- said device comprises a counter electrode (1) configured to contact said liquid sample;
- a first (9) and second (7) electrically conductive connection connectable to a voltage source is connected to said working and counter electrode, respectively.

29. The device according to item 28, wherein a second surface of said chamber is formed by an electrically conductive material forming the counter electrode.

30. The device according to item 28 or 29, wherein a third surface of said chamber is formed by an electrically conductive material forming a reference electrode.

31. The device according to any one of items 28 to 30, wherein the chamber forms a flow cell,
the flow cell comprising an inlet port and an outlet port separated by a cell volume, and the working electrode being positioned opposite the counter electrode between the inlet port and the outlet port.

32. The device according to any one of items 28 to 31, wherein the high-surface, electrochemically active material is selected from the group comprising carbon microfibers, metal microfibers, nanowires, nanopillars, micropillars, glassy carbon, screen-printed carbon, carbon films, carbon fibers, carbon paste, carbon nanotubes.

33. The device according to any one of items 28 to 32, wherein the high-surface, electrochemically active material is a carbon fibre (CF) microelectrode, particularly a carbon microfiber mesh, more particularly wherein the working and counter electrode and, optionally, a reference electrode, are formed by a carbon fibre microelectrode.

34. The device according to any one of items 28 to 33, wherein the working electrode and the counter electrode are separated by a distance ranging from 1 μm to 100 mm, particularly wherein the distance ranges from 10 μm to 10 mm, more particularly wherein the distance ranges from 50 μm to 1 mm.

35. The device according to any one of items 28 to 34, wherein the high-surface, electrochemically active material, particularly the CF microelectrode, is embedded in a polymer matrix, and a portion of the conductive micro- or nanostructured material is exposed to said liquid sample and or said loading solution,
particularly wherein the exposed portion protrudes 0.5 μm to 1 mm into the liquid sample.

36. The device according to any one of items 28 to 35, wherein the conductive micro- or nanostructured material, particularly the CF microelectrode, is characterized by any of the following parameters:

a. Density less than 1.5 g/$cm^3$, more particular: 1.1 - 0.1 g/$cm^3$,

b. Area density: 200 - 30 g/$m^2$,

c. In-plane electrical resistance (van der Pauw method): 1.5 - 0.015 Ω·mm.

d. Resistivity of $10^{-3}$ - $10^{-6}$ Q·m at 20 °C;

e. Suitable to perform electrochemical analysis (cyclic voltammetry) between -0.8 to +0.8 V (vs reference electrode).

37. The device according to any one of items 10 to 36 wherein said non-electrically conductive polymer matrix comprises, particularly consists of, a material selected from the group comprising polystyrene, polycarbonate, polyacrylate, poly-methyl methacrylate, polyethylene glycol diacrylate, polyethylene, polyurethane, acrylonitrile butadiene styrene, nylon (polyamides), polylactic acid, polybenzimidazole, polyethersulfone, polyoxymethylene, polyether ether ketone, polyetherimide, polyphenylene oxide, polyphenylene sulfide, polypropylene, polyvinyl chloride, polyvinylidene fluoride, polytetrafluoroethylene (Teflon).

38. The device according to any one of items 28 to 37, wherein the surface is coated with a material providing cationic moieties, particularly wherein the surface is coated with a material providing alkylamine moieties, more particularly wherein the surface is coated with an aminoalkylsilane, even more particularly wherein the surface is coated with 3-aminopropyl triethoxysilane (APTES), 3-aminopropyl methyl diethoxysilane (APMDES) or 3-aminopropyl dimethyl ethoxysilane (APDMES).

39. The device according to any one of items 28 to 38, wherein said conductive micro- or nanostructured material, particularly the CF microelectrode, is characterized by a ligand capable of specifically binding to said biomolecule being attached thereto, particularly wherein the ligand is covalently attached to the conductive micro- or nanostructured material, particularly the CF microelectrode.

40. The method according to any one of items 1 to 27, wherein the method is performed on a device according to any one of items 28 to 39.

The invention is further illustrated by the following examples and figures, from which further embodiments and advantages can be drawn. These examples are meant to illustrate the invention but not to limit its scope.

*Description of the Figures*

Fig. 1 -3 present a schematic illustration of an exemplary capturing and release system for EVs according to some embodiments of the present invention; 1- μCF counter electrode, 2- working electrode, 3- tubing, 4- fluidic channel, 5- current measurement, 6- PDMS matrix, 7- counter electrode contact, 8- Ag/AgCl applied area inside the fluidic channel, 9- working electrode contact, 10- embedded reference electrode in PDMS matrix, 11- reference electrode contact, 12- voltage control by the potentiostat.

Fig. 4-8 present a sketch with the dimensions of the aluminum mold that was used to fabricate the exemplary capturing and release system. The 3D Sketch (FIG. 4), top view - 58 mm-long fluidic channel (FIG. 5), cross-sections #1 and #7 from FIG. 5 (FIG. 6), cross-sections #2, #4 and #6 from FIG. 5 (FIG. 7), cross-sections #3 and #5 from FIG. 5 (FIG. 8). FIG. 7 and 8, the fluidic channel height is defined by the height of the step of the mold. 0.01 mm to generate a channel with a height of 0.2 mm, or 0.2 mm to generate a channel with a height of 0.6 mm.

Fig. 9 presents images of the fabrication process of an electrochemical fluidic channel, used as capturing and release system, according to some embodiments of the present invention; **A)** Image of an aluminum mold used to form the fluidic channel. **B)** Image of two aluminum molds with attached μCF by carbon double-sided tape: on the first mold, the two separated μCF pieces were placed on carbon tape with a distance of 3 mm. The larger (7 cm×1cm) μCF piece served as a working electrode (WE) while the smaller one (1.3 cm×1cm) served as a reference electrode (RE). After applying the double-sided carbon tape on the second aluminum mold, the μCF piece (7 cm×1 cm) aimed to serve as a counter electrode (CE), was perforated with needles, on which silicone tubes (3 mm inner diameter) were worn. **C)** Image of assembled mold, from the central upper view, with microscope glass slide between the molds. Both molds were covered with PDMS (curing agent at 10:1 mass ratio, vacuum pump removing the gas bubbles, then cured at 70° C (atmospheric pressure for 3 h) inside a vacuum oven. **D)** image of the final product of fabrication, which includes 2 semi channels. These semi-channels were removed gently from the mold after the PDMS was cured and the needles were removed. The upper semi channel includes the silicon tubing and the counter electrode (CE). The bottom semi-channel includes the working electrode (WE) and the reference electrode (RE) which was covered with Ag/AgCl paste.

Fig. 10. presents an image of the whole electrochemical fluidic channel which constructs the capturing and release unit, when connected to a potentiostat. **A and C)** A three-electrode cell is created inside the fluidic channel when the μCF pieces are connected via steel needles to the potentiostat's crocodile contacts. The steel needles are inserted into the PDMS by penetrating through the μCF pieces, which allows them to perform as electrodes in a fluidic cell. The red crocodile is connected to the working electrode (WE). The black crocodile is connected to the counter electrode (CE). The blue electrode is connected to the reference electrode (RE). The two parts of the fluidic channel are squeezed together between two polymethyl methacrylate (PMMA) lids with six bolts and six nuts. 1- μCF counter electrode, 2- working electrode, 3- tubing, 4- fluidic channel, 6- PDMS matrix, 7- counter electrode contact, 8- Ag/AgCl applied area inside the fluidic channel, 9- working electrode contact, 10- embedded reference electrode in PDMS matrix, 11- reference electrode contact.
**B)** The voltammetry characterization. The 5 repeated CV measurements, performed in PBS, are showing the stability of the electrochemical system that is located inside the fluidic channel (conditions: scan rate:0.02 V/s, step:0.002 V, $E_{begin}$= 0.0V, $E_{vertex1}$=0.1V, $E_{vertex2}$=-0.1 V).

Fig. 11. shows the top panel- SEM image (secondary electrons detector at 10 kV) of the μCF electrode used for integration into the fluidic channel. Bottom panel- a cross-section of the 8-mm wide fluidic channel with the μCF embedded into the PDMS as obtained by DMI6000 Leica microscope (Leica microsystems, Wetzlar, Germany).

Fig. 12. presents NTA analysis of EVs sample purified from urine by a device bearing a channel with a diameter of -600 μm and released into PBS. A) size distribution of the isolated EVs, as measured by NTA. Solid line- EVs sample purified from urine by anti-CD63 modified μCF fluidic channel. Dash-dotted line - control: urine sample processed by μCF fluidic channel that was not modified with antibodies. Dashed line- control: PBS buffer that was processed by an anti-CD63 modified μCF fluidic channel. B) A picture of urine sample following purification with our device. C) A table summarizing the size distribution of particles from the blue plot. This size distribution of 50-200 nm is typical for exosomes.

Fig. 13. presents SEM image of urinary EVs purified by our device and released into PBS. The purified sample was tipped on the μCF surface. The images were obtained by SEM secondary electrons detector at 5 kV. Nanoparticles of -150 nm can be observed on the fibers of the μCF.

Fig. 14. presents biomarkers analysis of the EVs isolated from urine. The left panel relates to samples purified by a CD9-modified device bearing a channel with a diameter of -600 μm, while the right panel relates to samples purified by a CD63-modified device. **A)** western blot (CD9 and albumin) and **B)** dot-blot analyses (TSG101, CD9, CD63, and Albumin), which compare the expression of exosomal biomarkers in purified EVs samples to their expression in the unprocessed urine sample. **C)** DLS measurement of a purified sample. **D)** Western blot analysis indicates the presence of the exosomal biomarker CD9 in the purified EVs sample.

Fig. 15. presents biomarkers analysis of the EVs isolated from urine, by a CD9-modified device bearing a channel with a diameter of -200 μm. **A)** dot-blot (TSG101, CD9, CD73, CD63, and Albumin) and **B)** western blot (CD63, CD9, and albumin) analyses, which compare the expression of exosomal biomarkers in purified EVs samples to their expression in the unprocessed urine sample.

Fig. 16. presents NTA analysis of EVs sample purified from urine by an anti-CD9 modified device bearing a channel with a diameter of -200 μm and released into PBSX0.001.

Fig. 17. presents an image of the whole device when connected to a MicroPulser electroporator (Bio-Rad Laboratories, Inc., 1000 Alfred Nobel Drive Hercules, CA, USA). the μCF pieces are connected via 4 steel needles (0.5 × 40 mm, Braun, B-Braun Melsungen AG) to the copper wires, which are connected via the black crocodile to the poles of the electroporator. One pole is connected to the working electrode, and the other pole is connected to the counter electrode. The reference electrode is disconnected.

Fig. 18. presents EMSA of the EVs isolated from urine and loaded with Cy5-siRNA. **Lane A)** control- 40 nM of Cy5-siRNA sample that was injected to our device and was subjected to 330 V for 1 ms for 8 times, similarly to electroporation but without the CD-9 electrode modification, indicating the migrated free Cy5-siRNA (lower bands). **Lane B)** EV sample loaded with Cy5-siRNA by electroporation using our device, indicates the presence of non-migrated loaded EVs (top band) along with free Cy5-siRNA (lower bands).

Fig. 19. presents NTA analysis of EVs sample purified from urine by an anti-CD9 modified device and loaded with Cy5-siRNA by a subsequent electroporation step. The right panel demonstrates the size distribution of isolated EVs before the loading step and the left panel demonstrates the size distribution of loaded isolated EVs. The distribution curve demonstrates a lower number of particles alongside increased diameter in the loaded EVs.

Fig. 20. presents images of HeLa cells transfected with EVs loaded with Cy5-siRNA by our device (lower panel) versus control- HeLa cells transfected with 40 nM of Cy5-siRNA sample that was injected to our device and was subjected to 330 V for 1 ms for 8 times, similarly to electroporation but without CD-9 electrode modification (upper panel).

Fig. 21. Is the enlargement of the dashed square from Fig. 20, right image lower panel, indicating the presence of Cy5 fluorescence (white arrows) inside the HeLa cells that were treated with the loaded EV solution.

Fig. 22. 100 μm step in PDMS semi channel which constracts the device with the 200 μm fluidic channel. Measured by Keyence scanning laser microscope

## Examples

### *Example 1: Materials and Methods*

The micro-carbon-fibers (μCF) electrode is made of Freudenberg H14 (22 cmx30 cm, catalogue number: 1590038) carbon paper (Brand: Freudenberg Performance Materials SE & Co. KG, Durham, NC, USA), with 0.150 mm thickness, obtained from FuelCellStore, (College Station, TX, USA). For a 40 mm-long fluidic channel, the working electrode bears an area of 3.2 $cm^2$, the counter electrode bears an area of 2.8 $cm^2$, and the reference electrode bears an area of 0.24 $cm^2$. For a 58-mm long fluidic channel, the working electrode bears an area of 4.64 $cm^2$, the counter electrode bears an area of 4.24 $cm^2$, and the reference electrode bears an area of 0.24 $cm^2$ Double-sided adhesive carbon tape (8 mmx20 m), catalogue number (AGG3939), was purchased from Agar Scientific, Ltd (Stansted, Essex, UK). Needles with 0.90 mm diameter (100 Sterican® ø 0.90×50 mm, 20 G×2", Braun Injekt®, Melsungen, Germany) were used to define the fluidic tubing (FIG. 9B). For the connection between the syringe and the tubing, needles (the sharp edge of the needle was removed) with 1.10 mm diameter were used (FIG. 10A and C). Aluminium molds (FIGs. 4-9A) were fabricated by a computer numerical control (CNC) machine. Syringes of 1 mL (Norm-Ject Plastic Syringe, Henke-Sass Wolf GmbH, Tuttlingen, Germany) and 20 mL (Braun Injekt®, Melsungen, Germany) were used to delivering the liquids through the fluidic part of the device. Microscope slides (26×76×1mm, Menzel X50, Thermo Fisher Scientific Inc., Waltham, MA, USA) were used for spatial separation between the two aluminium molds during PDMS curing (FIG. 9C)

3-Aminopropyldimethylethoxysilane (APDMES), catalogue number (S00750-5g), was purchased from Fluorochem, Ltd. (Hadfield, UK) Silver/silver chloride (60/40) paste used for screen printing (Ag-AgCl), catalogue number (901773-50G), was purchased from Merck KGaA (Darmstadt, Germany). Ethanol was obtained from Merck KGaA (Darmstadt, Germany). Phosphate-buffered saline, pH 7.4 (PBS) was obtained from Thermo Fisher Scientific (Waltham, MA, USA). To form the PDMS matrix, SYLGARD®184 silicon elastomeric kit (Dow Silicones Deutschland GmbH, Wiesbaden, Germany) was used. The tubings were made of silicone rubber holes, with 1 mm inner diameter, and 3 mm outer diameter, with a length of 4.5 cm. Millipore (Burlington, MA, USA) Milli-Q water (deionized water, 18 mega-ohms) was used in all experiments.

Primary mouse monoclonal antibodies, anti-CD63 (sc-5275), anti-CD9 (sc-13118), anti-TSG101 (sc-7964), and anti-GAPDH (sc-47724), as well as the western blotting luminol reagent, were purchased from Santa Cruz Biotechnology Inc. (Dallas, TX, USA). Anti-albumin (mouse monoclonal antibody, A6684-100UL) was purchased from Merck KGaA (Darmstadt, Germany). The secondary horseradish peroxidase (HRP)-conjugated polyclonal goat anti-mouse immunoglobulin was obtained from Dako Denmark A/S (Glostrup, Denmark). Immun-Blot PVDF membranes with a pore size of 0.2 μm were from Bio-Rad Laboratories Inc. (Hercules, CA, USA). Corning 96-well Clear Polystyrene Microplates were purchased from Corning Inc. (Corning, NY, USA).

Protein quantification: the total protein amount of the EVs was determined by the Micro bicinchoninic acid (BCA) assay according to the manufacturer's instructions (Thermo Fisher Scientific, Waltham, MA, USA). Briefly, lyophilized EVs samples were diluted with deionized water. EVs sample was mixed with Laemli buffer at 1/1 V/V %. 1-3 μL were added per well of a Corning 96-well Clear Polystyrene Microplate. After the addition of 200 μL Micro BCA reagent per well, the solutions were incubated at 37 °C for 2 h and the absorbance at 562 nm was measured using a Tecan Infinite M200 (Tecan Group Ltd., Männedorf, Switzerland).

Scanning electron microscope (SEM) images were taken using FEI (Hillsboro, Oregon, WA, USA) Quanta 200F environmental scanning electron microscope. The oxygen plasma treatment of the μCF electrode surface was performed by CY-P2L-B150 2L Plasma Cleaner (Zhengzhou CY Scientific instrument co., ltd, Zhengzhou, Henan, China). PDMS degassing and curing APDMES modification, and Ag/AgCl paste drying, were performed by the vacuum drying oven (Salvis AG, Lucern, Switzerland) and a diaphragm pump (Vacuubrand 696290, Vacuubrand GmbH, Wertheim am Main, Germany). The resistance of the electrodes and the quality of the contacts were measured by UNI-T UT61E Digital Multimeter (Uni-Trend Technology, Kowloon, Hong Kong). The preconcentration of the samples by lyophilization was performed with a freeze dryer ALPHA 2-4 LSC (Martin Christ Gefriertrocknungsanlagen GmbH, Osterode am Harz, Germany). For cyclic voltammetry measurements and electrochemical release of EVs, EmStat3 Blue potentiostat (PalmSens BV, Houten, Netherlands) was used.

*Example 2: Three-electrode fluidic-channel fabrication and characterization.*

A fluid-delivery device was fabricated from aluminium molds (FIG. 9) using flexible PDMS elastomer, double-sided carbon tape, and μCF. A three-electrode PDMS fluidic-channel cell of 58 mm long was used in all experiments apart from FIG. 12 and FIG. 14, which were generated using a device with 40 mm long channel. The carbon tape was cut to obtain a similar size as that of the channel (58 mmx0.8 mm) and pasted to the mold. Three sheets of μCF were cut in a rectangular form to form the electrodes: 70 mmx10 mm - working electrode, 70 mm×10 mm - counter electrode, 15 mmx10 mm - reference electrode. The counter electrode sheet was pasted to fully cover the channel (FIG. 9B). The working electrode sheet was pasted in a manner that allowed a 2 mm distance from the reference sheet electrode, and only 3 mmx0.8 mm of the reference electrode sheet was pasted to the carbon tape on the mold. After that, the upstream and downstream silicon tubes were 4.5 mm long and had a 1 mm inner diameter applied into the aluminium mold using syringe needles (FIG. 9B). A microscope slide (26×76×1 mm) was placed between the two molds and the 4 aluminium sheets were placed around the molds to create a cavity to contain the PDMS. This whole structure was supported by aluminium foil in order to prevent PDMS leakage (FIG. 9C). Then, the aluminium molds were covered by SYLGARD®184 (curing agent at a 10:1 mass ratio), degassed in a vacuum until the PDMS got clear, and incubated overnight at 60° C to form a PDMS matrix. The resulting device was bearing dimensions of 76×26×10 mm. Finally, the needles were taken out of the holes of the mold, and the cured PDMS structure was gently removed from the mold and the slides. The carbon tape adhesive residues were washed using ethanol. The resulting device had two semi-channels, from which one contains PDMS embedded μCF counter electrode with inlet and outlet tubing, while the other contains PDMS embedded μCF working electrode that is separated by 2 mm from the future reference electrode, which is then modified with Ag/AgCl paste (FIG. 9D).

*Example* 3a: *Surface activation*

All the glass dishes utilized in the modification procedures were washed with ethanol and dried in a vacuum oven at 80 °C for 30 min. Prior to surface modification, the semi-channels with the embedded μCF electrode were washed with -40 mL of ethanol and then with -40 mL of deionized water. Then, both parts were put into a vacuum oven (connected to a diaphragm pump) for 30 min at 80 °C for drying. In order to activate the μCF surface for the silanization procedure, plasma treatment was applied at 60 W for 3 min with a pressure of 30 Pa of oxygen gas. In order to create the amino-silane monolayer on the PDMS embedded μCF electrode, a gas phase chemical adsorption of APDMES was performed using a vacuum oven as following: The μCF electrodes were left to react with 150 μL APDMES (inside a 2 mL glass vial) for 16 h at 85 °C in a covered glass Petri-dish. The vacuum oven was pumped until the pressure gauge showed 0 Pa and then the pumping was stopped. This allowed the APDEMS vapors to saturate the oven chamber. After incubation of 16 h, the oven pump was turned on again to remove the APDMES vapor. Then, the vial with the APDMES was removed, and the glass Petri-dish with the embedded μCF electrodes was put back in the heated oven under vacuum and 100°C for an additional 2 h. All modified semi-channels with the embedded μCF electrodes, once obtained, were washed with ethanol (-50 mL for each μCF electrode) and dried at 80 °C in the vacuum oven. A fine layer of Ag-AgCl paste was applied on the surface of the reference sheet and dried at 60 °C for 30 min.

*Example 3b: specific ligand layer*

*Cross Linker Binding:*

The device was covered with 8.3% glutaraldehyde containing 12 mM sodium cyanoborohydride in phosphate buffer 10 mM, pH=8.5 (60 min, room temperature).

Thereafter, the device was washed with deionized water, acetone, isopropanol, and deionized water again.

*Assembly:*

The tubing and channel were washed with isopropanol and deionized water. The tubing was thereafter connected to a syringe pump, and the system was washed by introducing phosphate buffer (PB) (10 mM, pH=8.5).

*Antibody Immobilization:*

CA 15-3 igG (40 μl, 1 mg/ml) was mixed gently with 700 μl PB (10 mM, pH=8.5) containing 12 mM sodium cyanoborohydride. The antibody solution was introduced into the system (at 4° C, overnight about 16 h), and the tubing system was thereafter washed with PB (10 mM, pH=8.5) while keeping the surface under the channel always wet.

*Blocking:*

A blocking solution containing ethanolamine (100 mM) and 12 mM sodium cyanoborohydride in PB (10 mM, pH=8.5) was introduced into the system for 3 h at room temperature, at a flow rate of 50 μl/min. The system was thereafter washed with phosphate buffer (10 mM, pH=8.5) at a flow rate of 50 μl/min, for 30 min.

*Example 4: Fluidic electrochemical controlled channel assembly and characterization.*

Before assembling the two semi-channels, the conductivity of the electrode was tested by a multimeter. The conductivity of the electrode should be around 3 Ω/cm, which indicates that the surface of the electrode is highly conductive and can serve as an electrode. The two semi-channels have been adjusted together to form a fluidic channel. Then, these parts were squeezed together between two clear poly(methyl methacrylate) (PMMA) lids (each lid 105 mm×55 mm×5 mm), using six bolts and six nuts (FIG. 10). Afterward, steel needles were inserted into the PDMS, in a manner that the needles penetrated through the μCF pieces (without touching the fluidic part: the tubing and the channel), allowing them to perform as current collectors for the electrodes, inside a fluidic electrochemical cell. The fluidic system was washed with 30 mL of deionized water and then with 30 mL of phosphate-buffered saline (PBS) ($KH_2PO_4$-1 mM, NaCl-155 mM, $Na_2HPO_4$-$7H_2O$-3 mM, pH 7.4). Then, the metal needles were connected to the potentiostat according to FIG. 10. the electrochemical cell inside the fluidic channel was characterized by cyclic voltammetry (CV) in order to test the stability of the system. The 5 repeated CV measurements in PBS are presented in FIG. 10B (conditions: scan rate: 0.02 V/s, step:0.002 V, $E_{begin}$=0.0 V, $E_{vertex1}$=0.1 V, $E_{vertex2}$=-0.1 V). Immediately after CV analysis, the fluidic channel was immobilized with anti-CD63 or anti-CD9 (an antibody against CD63 or CD9, which are present on the surface of exosomes), by adding 32 μL (concentration 200 μg/mL) of the antibody solution into 250 μL PBS and injecting it into the fluidic channel, using a 1 mL syringe. On the other end of the fluidic channel, another empty syringe of 1 mL was used to collect the solution. The antibody solution was transferred between the two syringes for 1.5 h. Then 15 mL of PBS (at a flow rate of 0.3 mL/s) was injected through the channel in order to clean the channel from the unadsorbed antibodies.

*Example 5: Sample purification:*

Samples of 50 to 200 mL of human urine (Human Urine Male, BioIVT, West Sussex, United Kingdom) were injected at a rate of 0.3 to 1 mL/s through the μCF fluidic channel. Then, 15 mL of PBS were injected at a rate of 0.3 mL/s through the μCF fluidic channel, to remove contaminants that were attached non-specifically to the modified fluidic system. For western blot and dot blot analyses, an additional 0.5 mL of 0.001xPBS was injected in the device at 0.3 mL/s in order to reduce the salts concentration. During lyophilization, the salt concentration increases dramatically, which may affect the western blot analysis.

Before the electrochemical release, a syringe of 1 mL with 250 μL of PBS (or 0.001×PBS for western blot analysis) was connected to one end of the device, and another empty 1 mL syringe was connected to the second end of the device. Then, the needles of the device were connected to the potential source as shown in FIG. 10. The potential on the working electrode was set to -1.6 V for 18 s in order to release the EVs from the electrode surface. During this time, the PBS was mixed inside the channel using the syringe with a flow rate of 110 μL/s. Immediately after the release, all the liquid from the device was withdrawn into one of the syringes and taken for further analysis.

*Example 6: Sample electroporation for siRNA loading*

The fluidic channel was assembled as described at the section entitled "Fluidic electrochemical controlled channel assembly and characterization". The channel was immobilized with anti-CD9 antibody by adding 32 μL (concentration 200 μg/mL) of the antibody solution into 500 μL PBS and injecting it into the fluidic channel, using a 1 mL syringe. On the other end of the fluidic channel, another empty syringe of 1 mL was used to collect the solution. The antibody solution was transferred between the two syringes for 1 h. Then 15 mL of PBS (at a flow rate of 0.3 mL/s) was injected through the channel in order to clean the channel from the unadsorbed antibodies. Then, a sample of 150 mL of human urine (Human Urine Male, BioIVT, West Sussex, United Kingdom) was injected at a rate of 0.3 to 1 mL/s through the μCF fluidic channel. Then, for the subsequent loading step, a solution of 10 μL of Cy5-siRNA (20 μM) in 200 μL of PBS was injected into the fluidic channel, using a 1 mL syringe. On the other end of the fluidic channel, another empty syringe of 1 mL was used to collect the solution. The Cy5-siRNA solution was transferred between the two syringes for 30 min. 1 mL of deionised water (DIW) were injected at a rate of 0.3 mL/s through the μCF fluidic channel, to remove non-attached Cy5-siRNA. Electroporation was performed by applying 330 V for 1 ms for 8 times, followed by a release step by applying -1.7 V for 90 s.

*SEM Analysis*

EVs from 50 mL of urine were purified and released in PBS. Then 10 μL of the purified urinary EVs were dropped on a piece of 1 cmx1 cm μCF. The EVs were detected by a secondary electron detector. 70 different EVs were counted and measured by ImageJ software (National Institutes of Health, MD, USA). The average measured diameter of the EVs was 140 ±60 nm.

*Protein quantification*

In order to quantify the amount of purified EVs, a micro BCA assay was applied. According to this assay, the amount of EVs protein per mL, released from a device bearing a 58-mm long channel into 0.5 mL, was 2-150 μg/mL. According to (Liu et al., 2018), 1 mL of urine sample contains 7.5*$10^9$ EVs. According to (Eugene D. Sverdlov, 2012), 1 μg EV protein corresponds to 2 *$10^9$ vesicles. Therefore, 1 mL of urine has 3.75 μg of EV protein. Finally, the enrichment factor is estimated to be 0.**1-13%**

*Dot blot analysis*

Samples of isolated EVs were lyophilized and concentrated by dissolving in 1/10 of the initial volume. Concentrated EVs samples were evaluated for protein's content by micro BCA assay (FIG. 14 left panel). Protein concentration was compared with a solution of liophilized urine in DDW. One μL was dropped on a nitrocellulose membrane, blocked with 5% skim milk, and reacted with the indicated primary antibodies (mouse), and then with secondary goat anti-mouse-HRP antibody. Membranes were reacted with western blotting luminol reagent and imaged using the ChemiDoc instrument (BioRad, Hercules, CA, USA).

*Western blot analysis*

Samples of isolated EVs were lyophilized and concentrated by dissolving in 1/5 of the initial volume. Concentrated EVs samples were evaluated for protein's content by micro BCA assay. 10 μg protein of concentrated EVs sample or liophilized urine dissolved in DDW were diluted 5:1 v/v in reducing sample buffer (0.25 m Tris, 10% w/v sodium dodecyl sulfate (SDS), 30% v/v glycerol, 0.02% w/v bromophenol blue, 5% v/v $\beta$-mercaptoethanol) and heated at 95 °C for 10 min. Proteins were resolved on 12% SDS polyacrylamide gels and transferred on 0.2 μm pore-sized Immun-Blot PVDF membranes. The membrane was blocked with 5% w/v skim milk powder dissolved in Tris-buffered saline (TBS-T, 20 mM Tris, 150 mM NaCl, 1% v/v polysorbate 20) for 1 h at room temperature (RT). The membrane was probed with primary antibodies, overnight at 4 °C. Then, the membrane was washed three times for 10 min each with TBS-T and incubated with the secondary horseradish peroxidase (HRP)-conjugated polyclonal goat anti-mouse immunoglobulin for 2 h at RT. The membrane was again washed three times for 10 min each with TBS-T and incubated with Western Blotting Luminol Reagent for 2 min at RT. Protein bands were imaged using a ChemiDoc instrument (BioRad, Hercules, CA, USA).

*Electrophoretic mobility shift assay (EMSA)*

Samples of 30 μL of EVs freshly isolated and loaded with Cy5-siRNA by our device were added with 5 μL of glycerol solution (50% V/V in DIW), and loaded on an agarose gel (1% W/V in TAE buffer). A voltage of 100 mV was applied for 30 min. Then, the gel was imaged for Cy5 fluorescence using a ChemiDoc instrument (Bio-Rad, Hercules, CA, USA).

*Dynamic light scattering (DLS) analysis*

DLS measurements were performed with a ZetaSizer Pro and presented as number-based distribution (Malvern Instruments, Malvern, Worcestershire, UK). Samples were not diluted and were tested in PBS at a total volume of 0.2 mL. automatic measurement runs were performed, with standard settings for liposome particles in water at RT.

*Nanoparticle tracking analysis (NTA) analysis*

The size profile, the images, and the concentration of EVs isolated from urine were analyzed on a ZetaView (Particle Metrix GmbH, Inning am Ammersee, Germany) equipped with a Complementary Metal Oxide Semiconductor camera and a 405 nm laser source. Samples were diluted in PBS 1:10 immediately after EVs were isolated. For the ZetaView, the sensitivity was set to 85, the shutter to 150, and the frame rate to 30. Data were analyzed with the ZetaView software version 8.04.04 SP2 applying a bin class width of 5 nm, minimum brightness of 25, minimum area of 5, maximum area of 1000, and trace length of 15. 87.5% of the purified EVs were in the range of 45-195 nm.

*Cell transfection*

Human cervical carcinoma (HeLa) cells were obtained from the American Tissue Culture Collection (ATCC). HeLa cells were cultured in DMEM supplemented with 10% fetal bovine serum (FBS), 100 U/mL Penicillin, 100 μg/mL Streptomycin, 12.5 U/mL nystatin. Cells were grown at 37 °C; 5% CO2. Cells were seeded at 200,000 cells/well in a 24 well plate, and incubated at $37^0$ C and 5% $CO_2$ for 3 h. Then, the medium was replaced with 300 μL of fresh medium, 80 μL of loaded exosomes solution/control solution was added. Following 6 h, the medium was replaced with PBS, and the cells were imaged on a Leica fluorescent microscope (CTR6000, Leica camera AG, Wetzlar, Germany).

## Claims

1. A method for capturing a biomolecule, wherein the biomolecule is presented on an extracellular vesicle, or for isolating an extracellular vesicle, said method comprising:

    a. in a binding step, contacting a liquid sample comprising said biomolecule with a surface, wherein

        i. said surface is electrically conductive,
        ii. and

            - said surface carries a chemical modification facilitating retention of said biomolecule, or
            - an electrical retention potential is applied to said surface, facilitating retention of said biomolecule;

    b. in a washing step, removing said liquid sample;
    c. in a release step, applying a release voltage to said surface,
    d. collecting the biomolecule;

    **characterized in that**
    said surface is an electrically conductive micro- or nanostructured material.

2. The method according to claim 1, wherein the surface comprises a ligand capable of specifically binding to said biomolecule.

3. The method according to claim 1 or 2, particularly wherein the biomolecule is presented on an exosome, and wherein

    a. in a loading step conducted subsequent to the binding step, and prior to the release step, the surface is contacted with a loading solution comprising a cargo molecule,
    b. a loading voltage having a polarity opposite to a polarity of the release voltage is applied to the surface.

4. A method for loading an extracellular vesicle with a cargo molecule selected from the group comprising a pharmaceutical drug, a protein, a nucleic acid, a dye molecule, comprising the steps:

    a. providing an aqueous medium comprising an extracellular vesicle, wherein the aqueous medium is in contact with a surface that is electrically conductive, wherein the aqueous medium is a loading solution comprises a cargo molecule;
    b. applying a loading voltage to the surface;

    **characterized in that**
    said surface is an electrically conductive micro- or nanostructured material.

5. The method according to any one of the preceding claims, wherein the conductive micro- or nanostructured material is a carbon fibre (CF) microelectrode, particularly a carbon microfiber mesh.

6. The method according to any one of the preceding claims, wherein the conductive micro- or nanostructured material, particularly the CF microelectrode, is embedded in a polymer matrix, and a portion of the conductive micro- or nanostructured material is exposed to said liquid sample and or said loading solution,
    particularly wherein the exposed portion protrudes 0.5 μm to 1 mm into the liquid sample.

7. The method according to any one of the preceding claims 1 to 6, wherein the conductive micro- or nanostructured material, particularly the CF microelectrode, is **characterized by** any of the following parameters:

    a. Density less than 1.5 g/$cm^3$, more particular: 1.1 - 0.1 g/$cm^3$,
    b. Area density: 200 - 30 g/$m^2$,
    c. In-plane electrical resistance (van der Pauw method): 1.5 - 0.015 Ω·mm.
d. Resistivity of $10^{-3}$ - $10^{-6}$ Q·m at 20 °C;
e. Suitable to perform electrochemical analysis (cyclic voltammetry) between -0.8 to +0.8 V (vs reference electrode).

**8.** The method according to any one of the preceding claims, wherein the ligand is capable of specifically binding to

a. an exosome-specific marker, particularly a marker selected from CD9, CD63, CD81, CD82, and CD86, or to
b. a non-specific surface protein selected from CD16, CD18, CD14, CD13, MCT-1, Na/K ATPase, Cd11b/Mac-1, MHC I and II, IL-1β, Flotillin-1, an integrin, an annexin, or a lipid-raft sphingolipid, cholesterol, or a ceramide.

**9.** The method according to any one of the preceding claims, wherein

a. the release voltage ranges from -3 V to 3 V, particularly from 0 V to -3 V, or from 0 V to 3 V;
b. the loading voltage ranges from -350 V to -50 V or from +350 V to +50 V, particularly wherein the loading voltage ranges from -300 V to -100 V or from +300 V to +100 V;
c. the loading voltage is applied as a loading voltage burst for 0.5 ms to 5 ms, particularly for about 1 ms;
d. the retention voltage ranges from +3 V to -3 V, particularly from +2 V to -2 V, even more particularly from +1.5 V to -1.5 V, yet even more particularly from +1 V to -1 V.

**10.** A device for isolating of a biomolecule, or for isolating and/or loading of an extracellular vesicle, said device comprising a chamber configured for receiving a liquid sample, wherein

- a first surface of said chamber is formed by a high-surface, electrochemically active material embedded in a non-electrically conductive polymer matrix, said first surface forming a working electrode (2);
- said device comprises a counter electrode (1) configured to contact said liquid sample;
- a first (9) and second (7) electrically conductive connection connectable to a voltage source is connected to said working and counter electrode, respectively;

wherein the high-surface, electrochemically active material is a carbon fibre (CF) microelectrode, particularly a carbon microfiber mesh, more particularly wherein the working and counter electrode and, optionally, a reference electrode, are formed by a carbon fibre microelectrode.

**11.** The device according to any one of claim 10, wherein the chamber forms a flow cell, the flow cell comprising an inlet port and an outlet port separated by a cell volume, and the working electrode being positioned opposite the counter electrode between the inlet port and the outlet port.

**12.** The device according to any one of claims 10 or 11, wherein the working electrode and the counter electrode are separated by a distance ranging from 1 μm to 100 mm, particularly wherein the distance ranges from 10 μm to 10 mm, more particularly wherein the distance ranges from 50 μm to 1 mm.

**13.** The device according to any one of claims 10 to 12, wherein the high-surface, electrochemically active material, particularly the CF microelectrode, is embedded in a polymer matrix, and an exposed portion of the conductive micro- or nanostructured material is exposed to said liquid sample and or said loading solution,
particularly wherein the exposed portion protrudes 0.5 μm to 1 mm into the liquid sample.

**14.** The device according to any one of claims 10 to 13, wherein the conductive micro- or nanostructured material, particularly the CF microelectrode, is **characterized by** any of the following parameters:

a. Density less than 1.5 g/$cm^3$, more particular: 1.1 - 0.1 g/$cm^3$,
b. Area density: 200 - 30 g/$m^2$,
c. In-plane electrical resistance (van der Pauw method): 1.5 - 0.015 Ω·mm.
d. Resistivity of $10^{-3}$ - $10^{-6}$ Ω·m at 20 °C;
e. Suitable to perform electrochemical analysis (cyclic voltammetry) between -0.8 to +0.8 V (vs reference electrode).

**15.** The device according to any one of claims 10 to 14, wherein said conductive micro-or nanostructured material, particularly the CF microelectrode, is **characterized by** a ligand capable of specifically binding to said biomolecule being attached thereto,
particularly wherein the ligand is covalently attached to the conductive micro- or nanostructured material, particularly the CF microelectrode.

FIG. 1

Top View

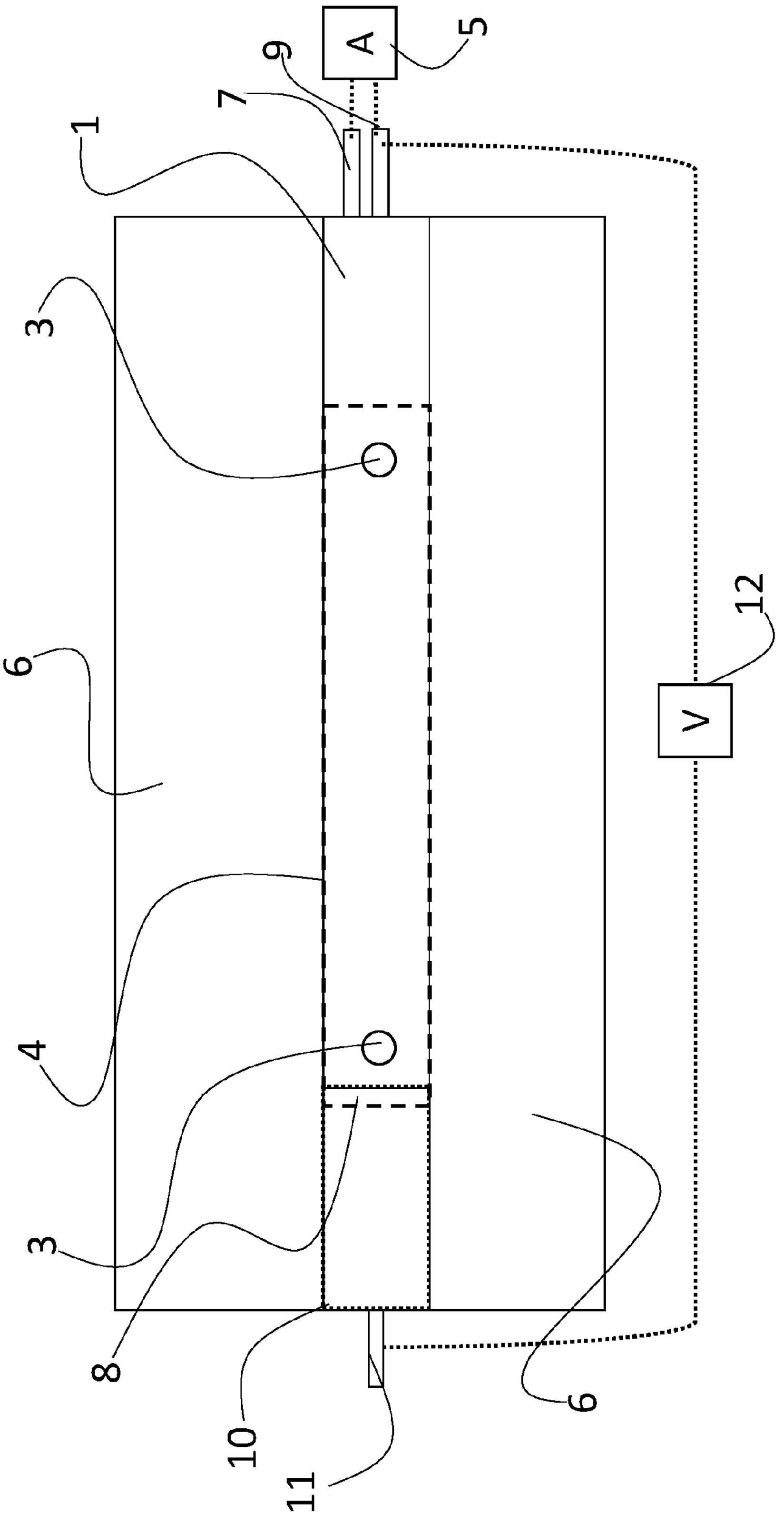

Cross section view 1

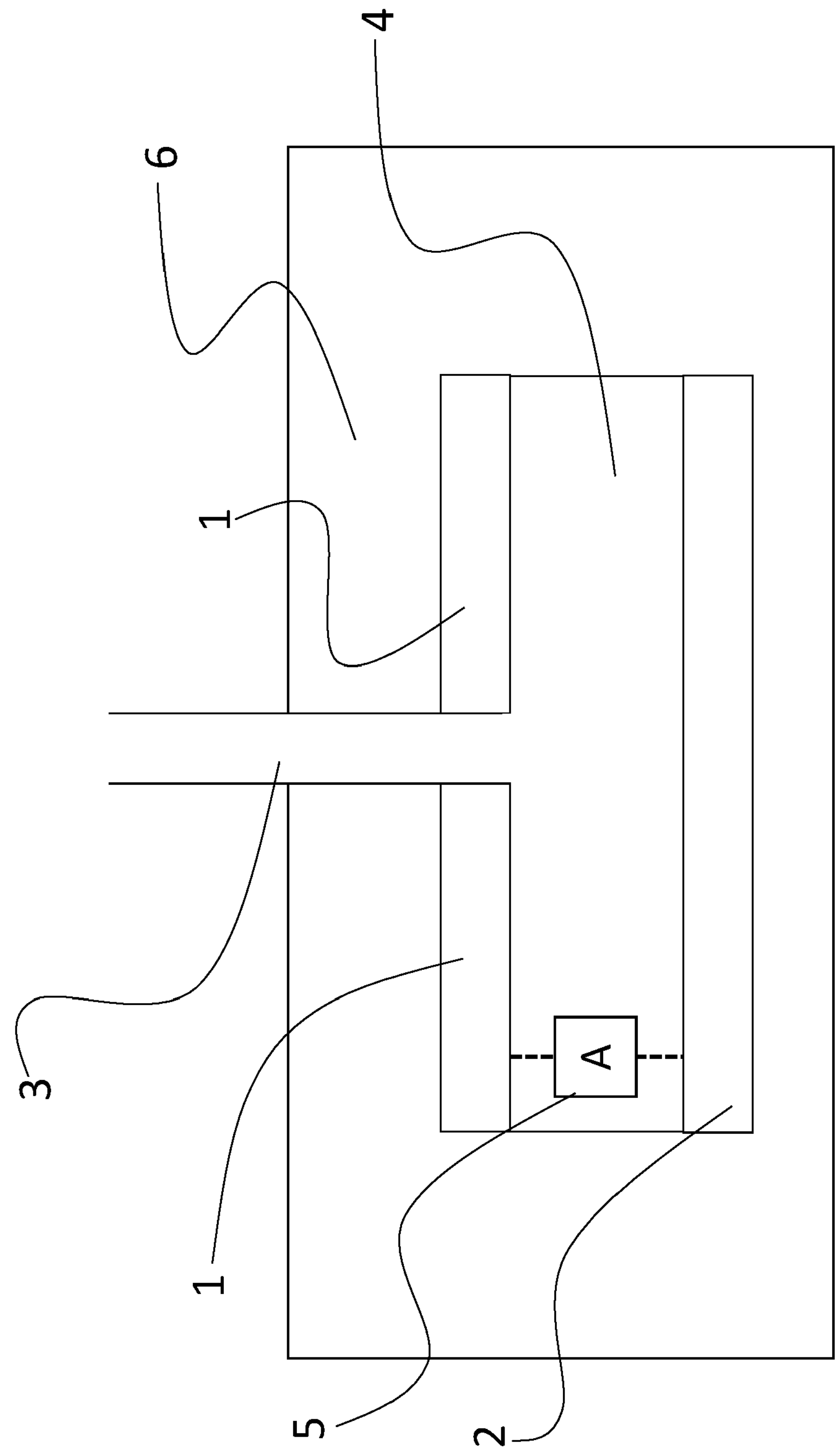


FIG. 2

FIG. 3

Cross section view 2

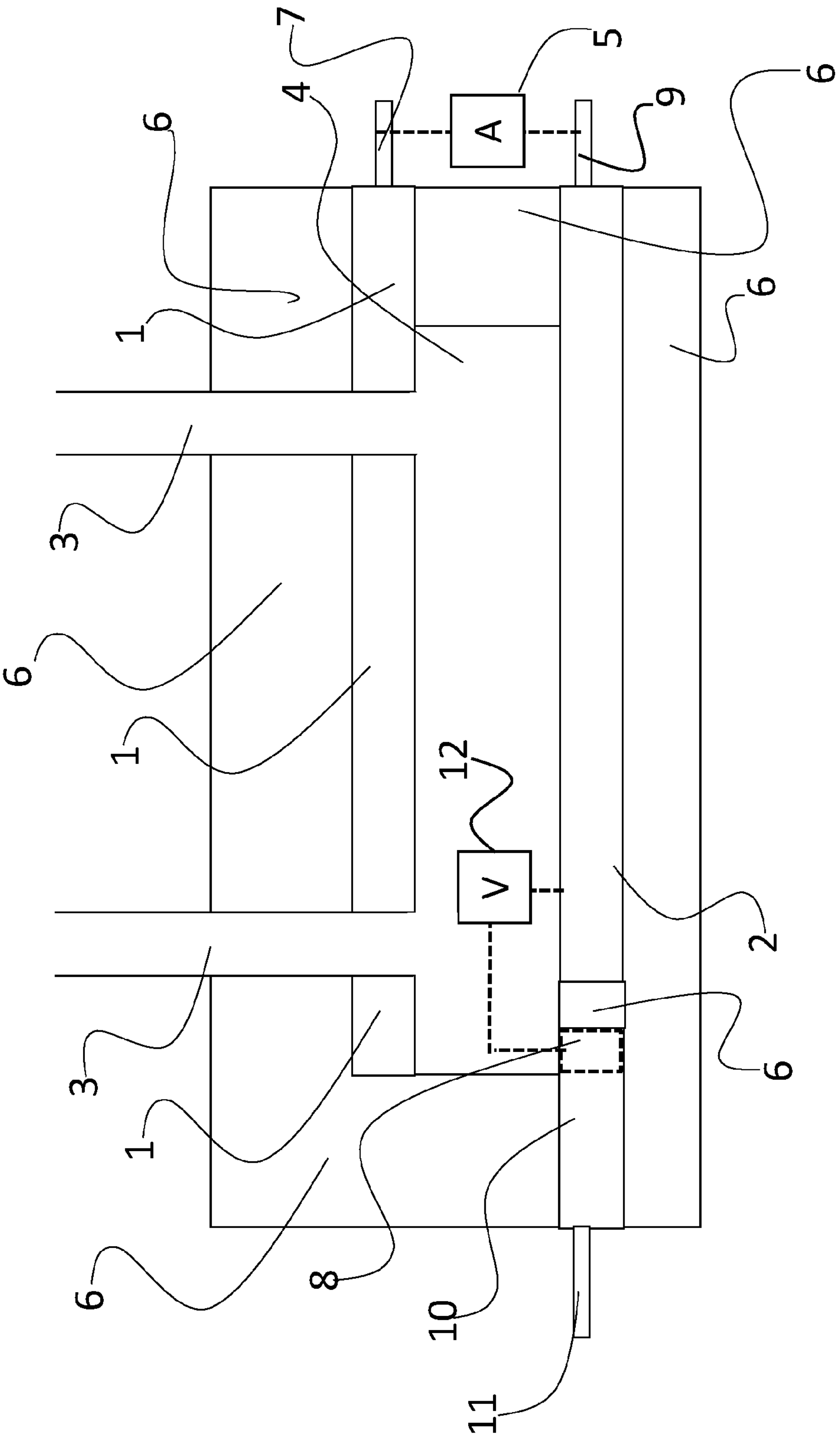

3D sketch

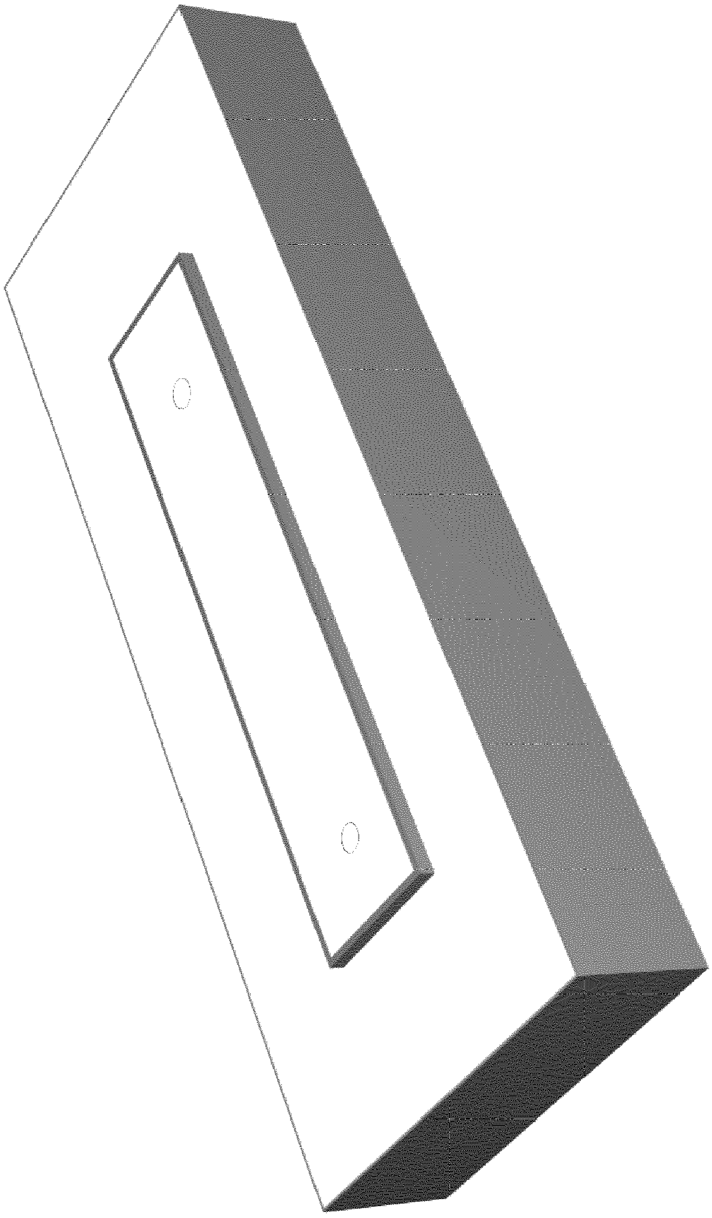

FIG. 4

FIG. 5

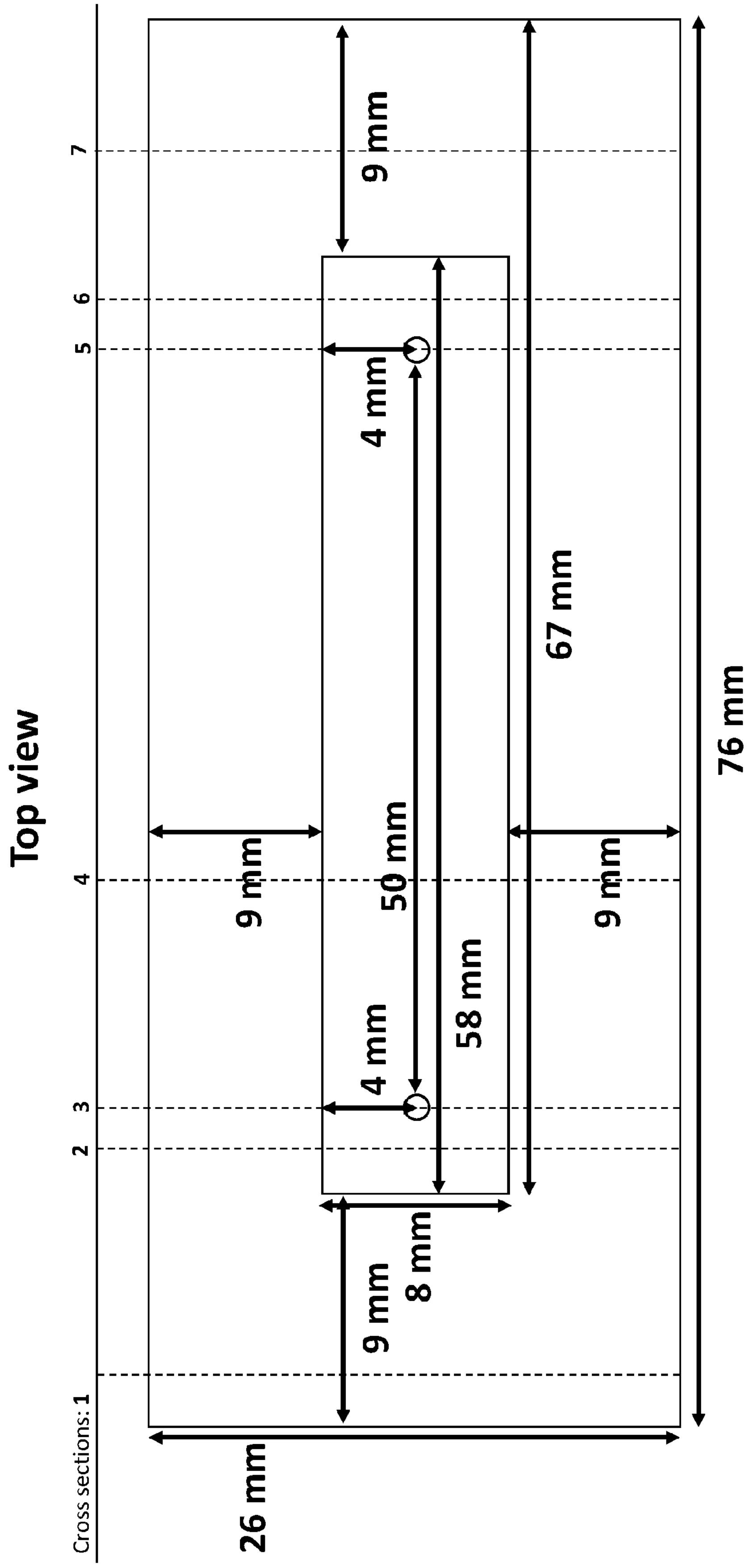
Top view
Cross sections: 1
2
3
4
5
6
7
26 mm
9 mm
8 mm
4 mm
58 mm
50 mm
9 mm
9 mm
4 mm
67 mm
76 mm
9 mm

FIG. 6

Cross section #1, and #7

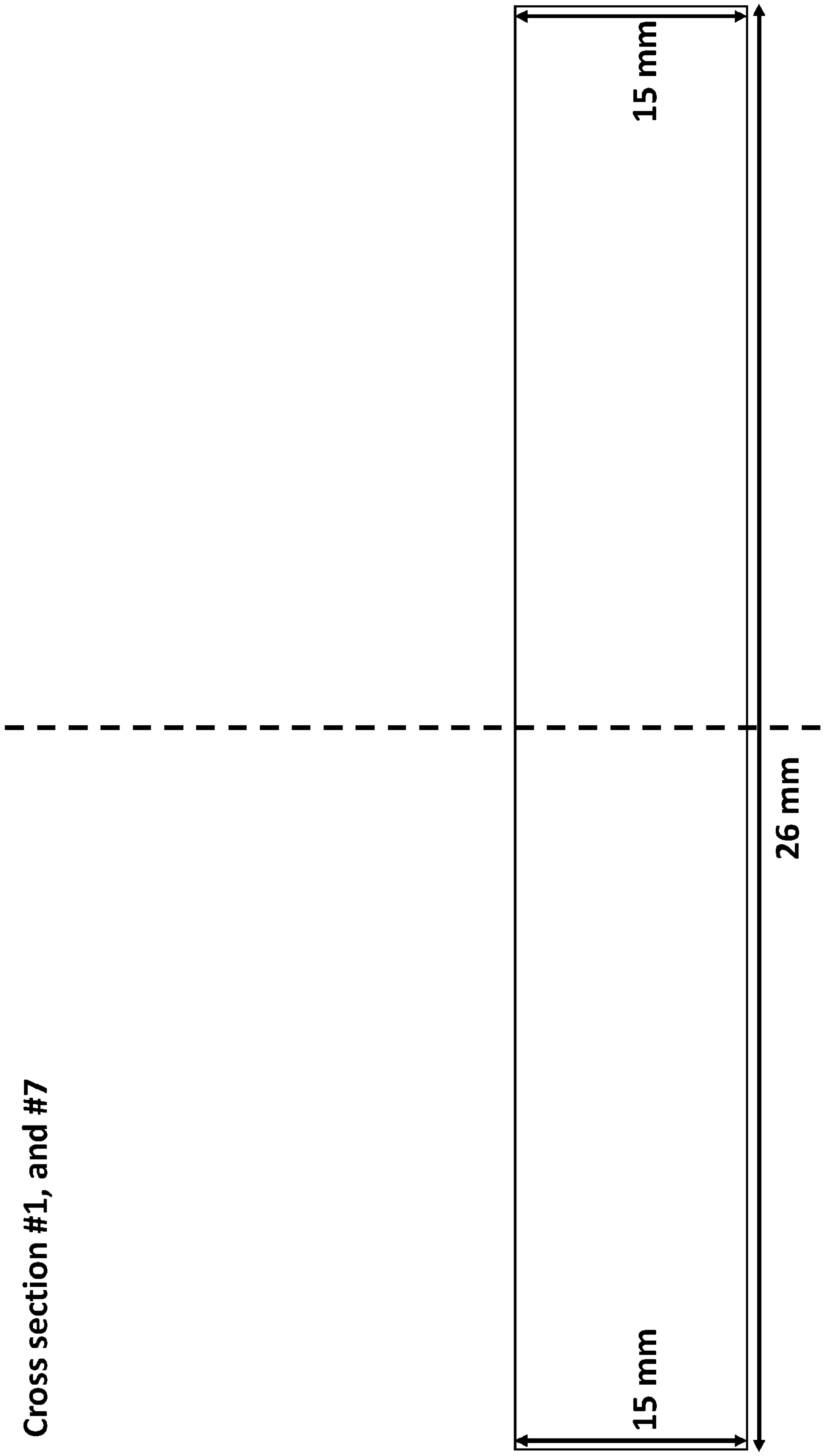

FIG. 7

Cross section #2, #4 and #6

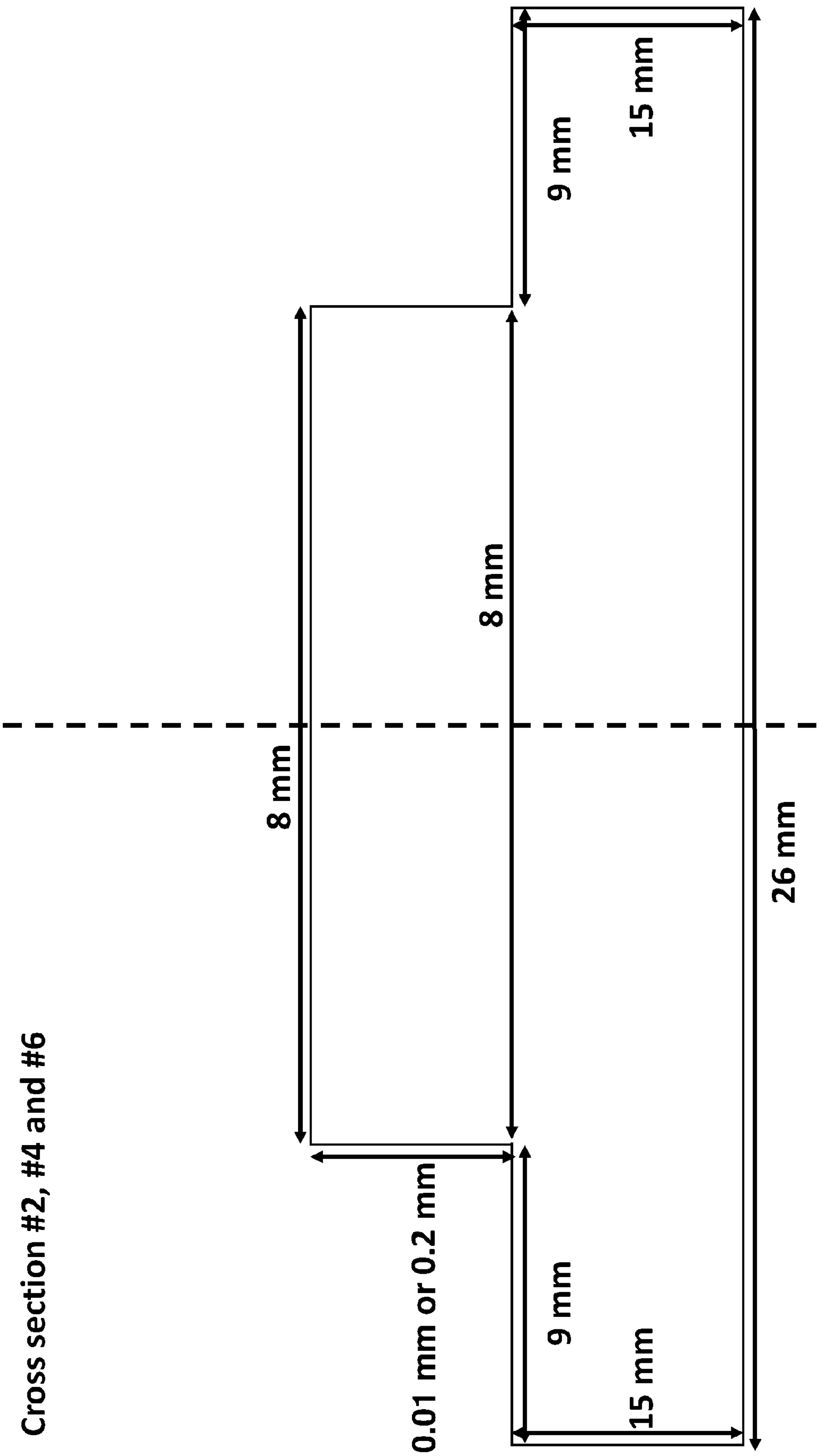

FIG. 8

Cross section #3 and #5
Note: round hole with a diameter of 1 mm

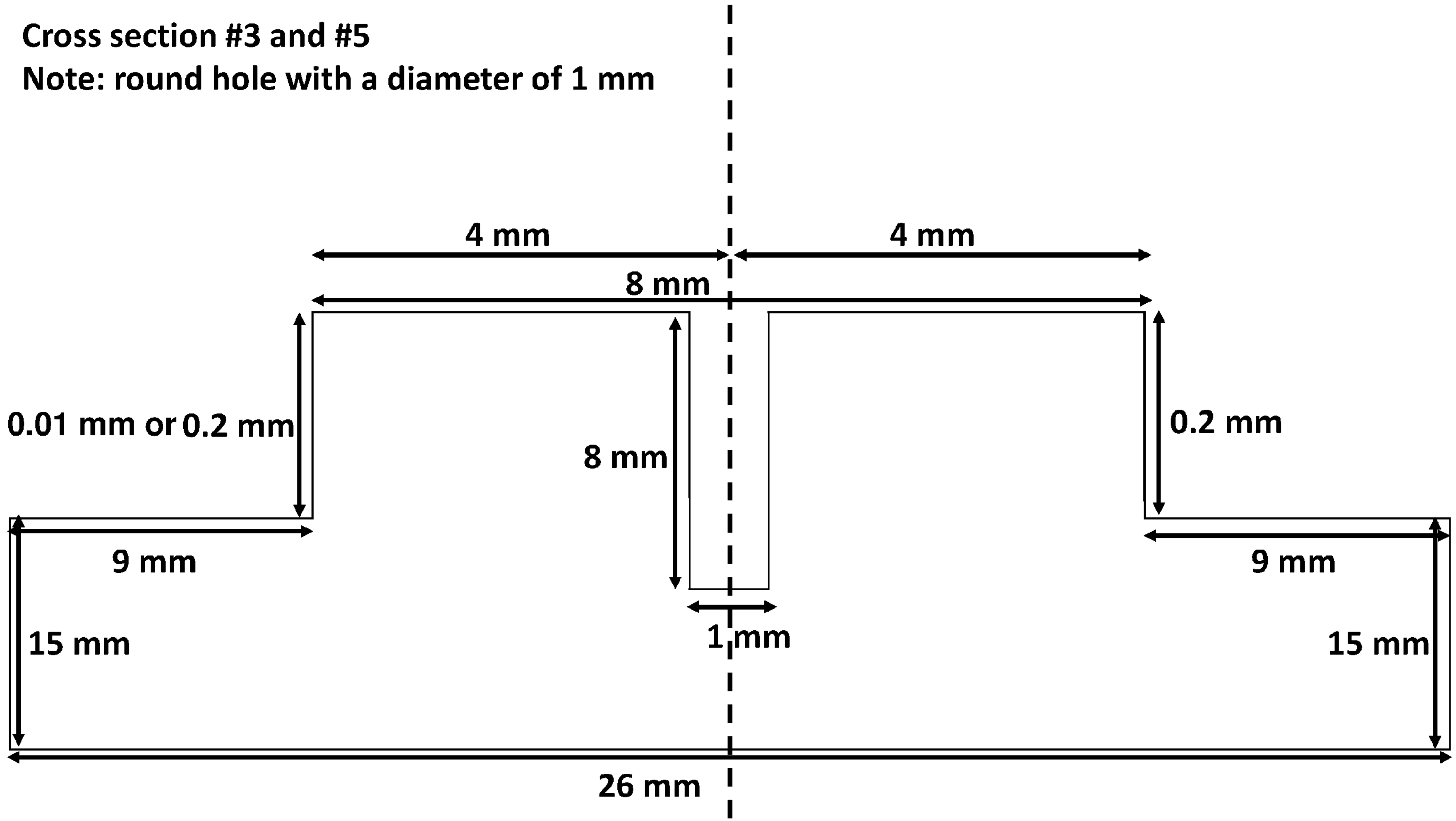

FIG. 9

Device Fabrication

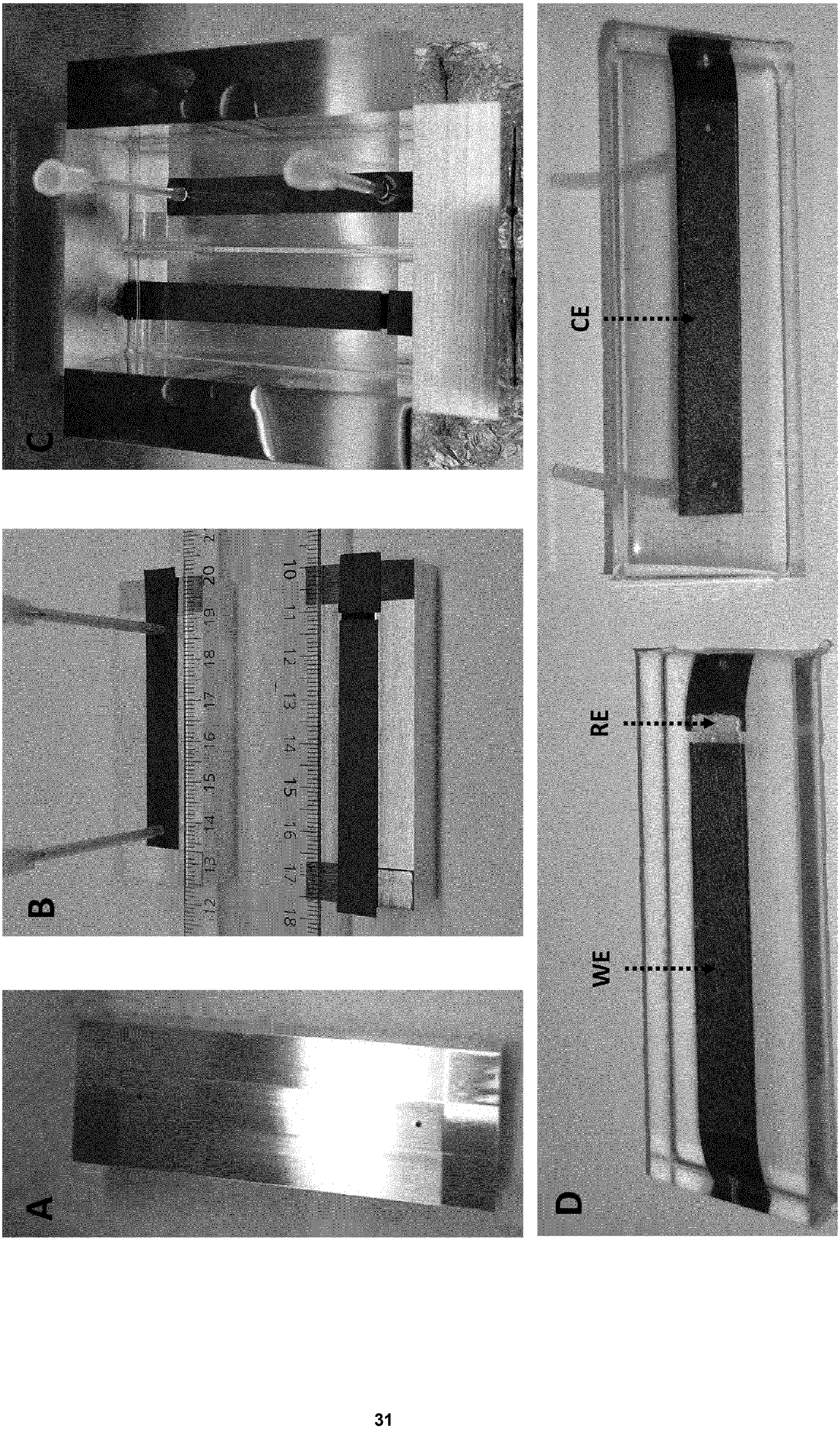

FIG. 10

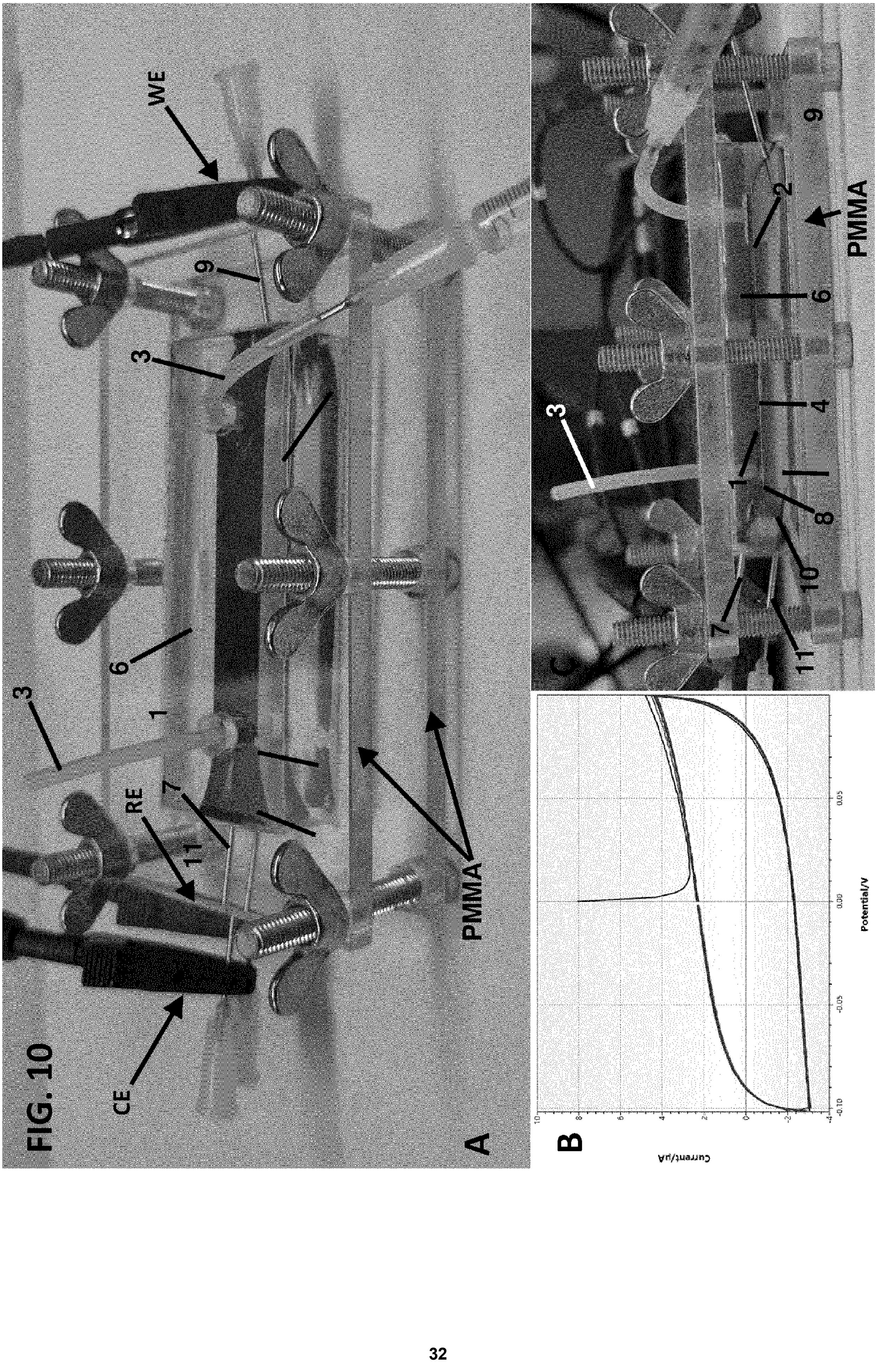
A
CE
RE
WE
PMMA
1
3
6
7
9
11
B
Current/µA
Potential/V
C
1
2
3
4
6
7
8
9
10
11
PMMA

FIG. 11

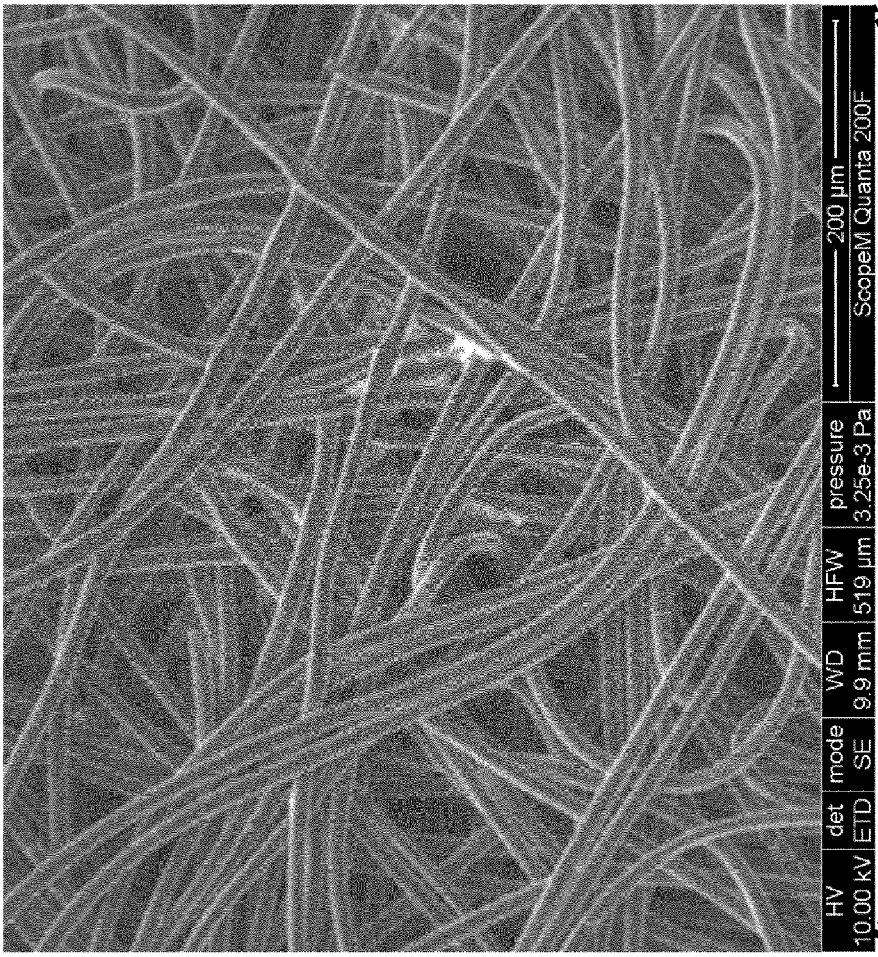
HV | det | mode | WD | HFW | pressure | 200 µm
10.00 kV | ETD | SE | 9.9 mm | 519 µm | 3.25e-3 Pa | ScopeM Quanta 200F

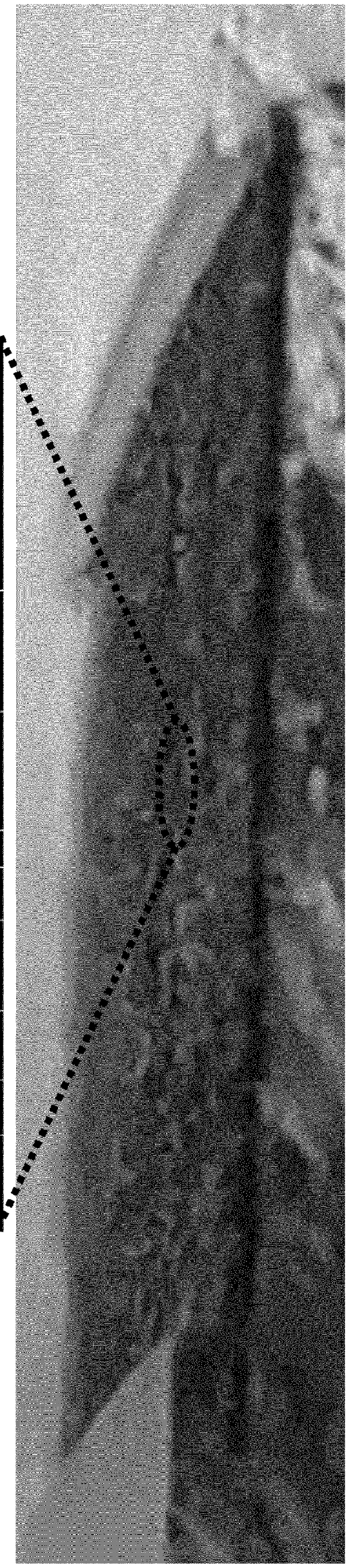

FIG. 12

A

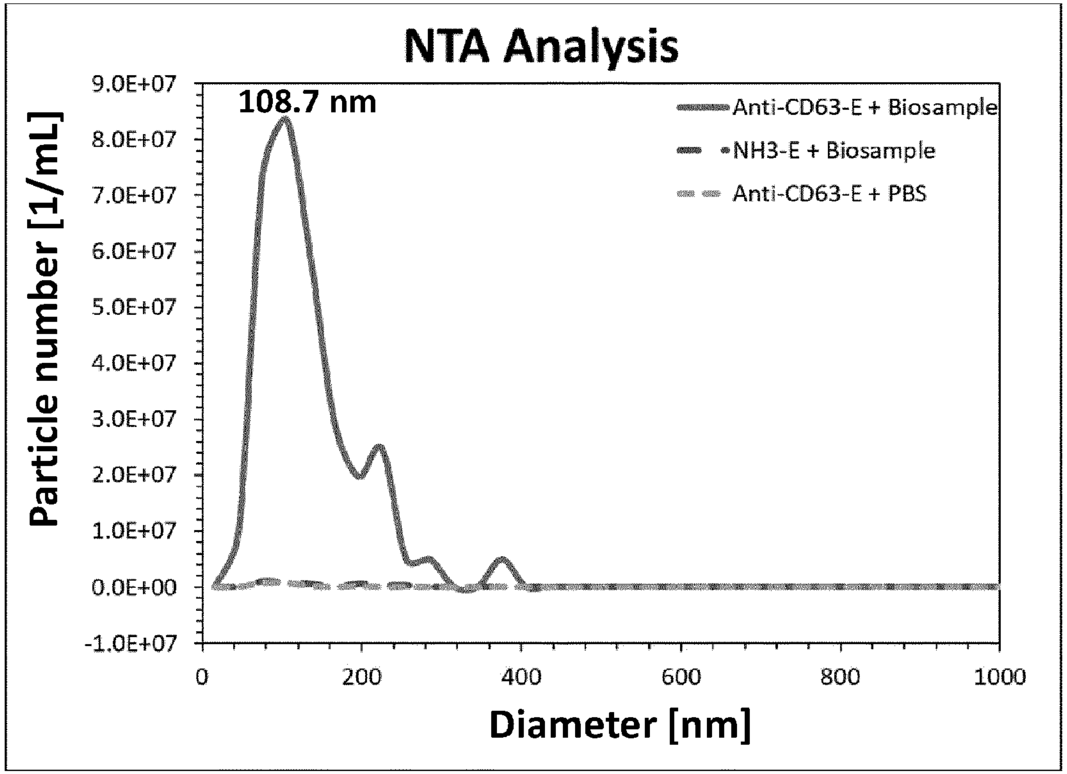


B

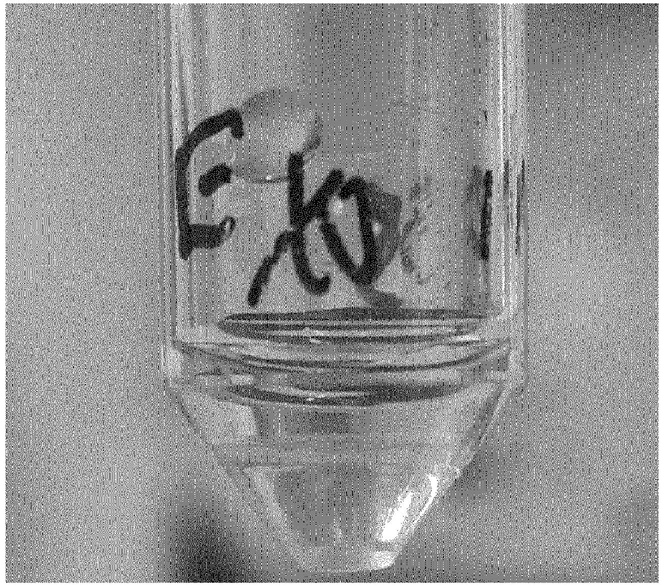

C

| EVs 45-165 nm | EVs 45-195 nm | Total |
|---|---|---|
| **81.3 %** | **87.5%** | **100%** |
| 2.56E+08 | 2.76E+08 | 3.15E+08 |

FIG. 13 Scanning electron analysis of purified exosomes on µCF

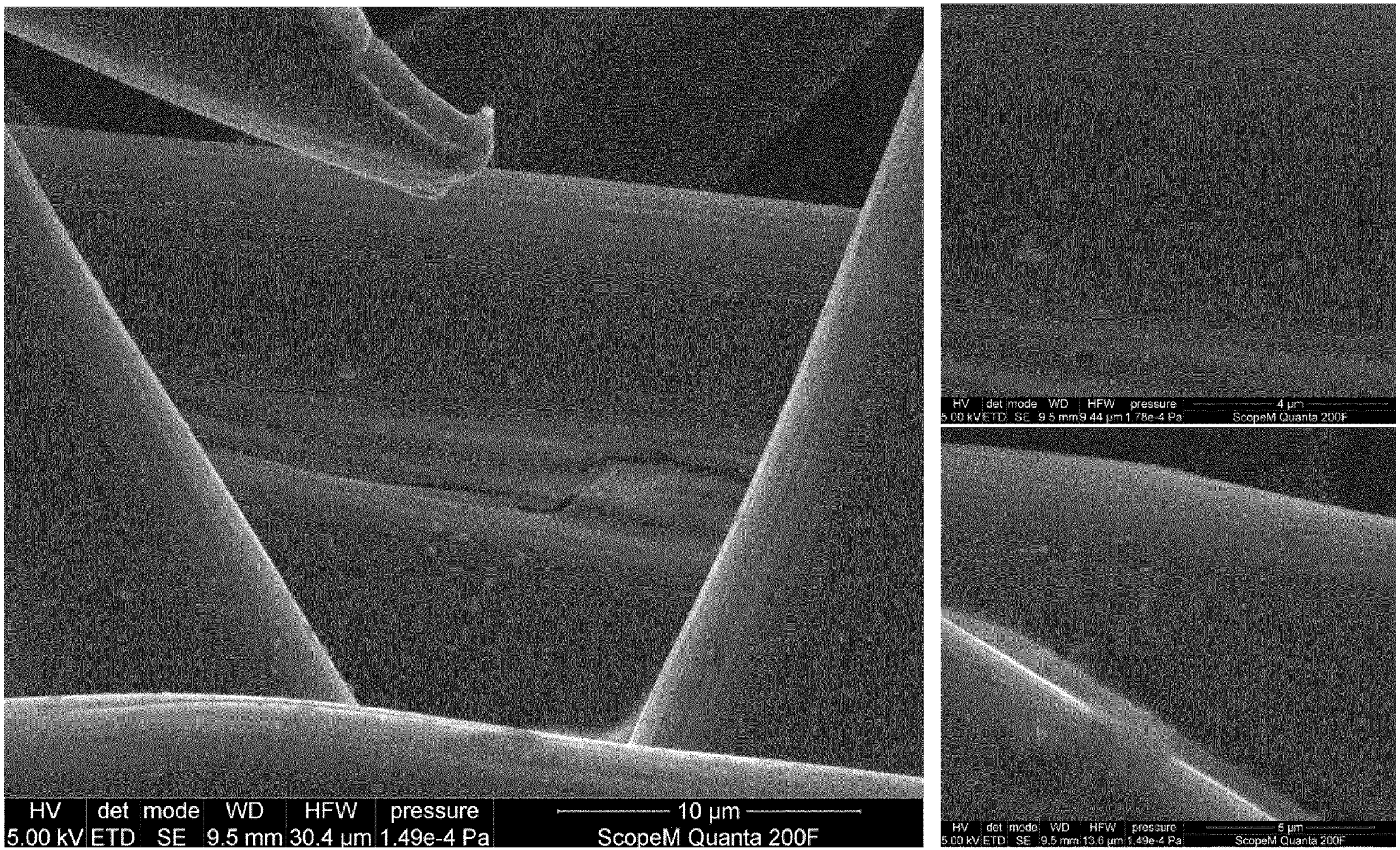

FIG. 14

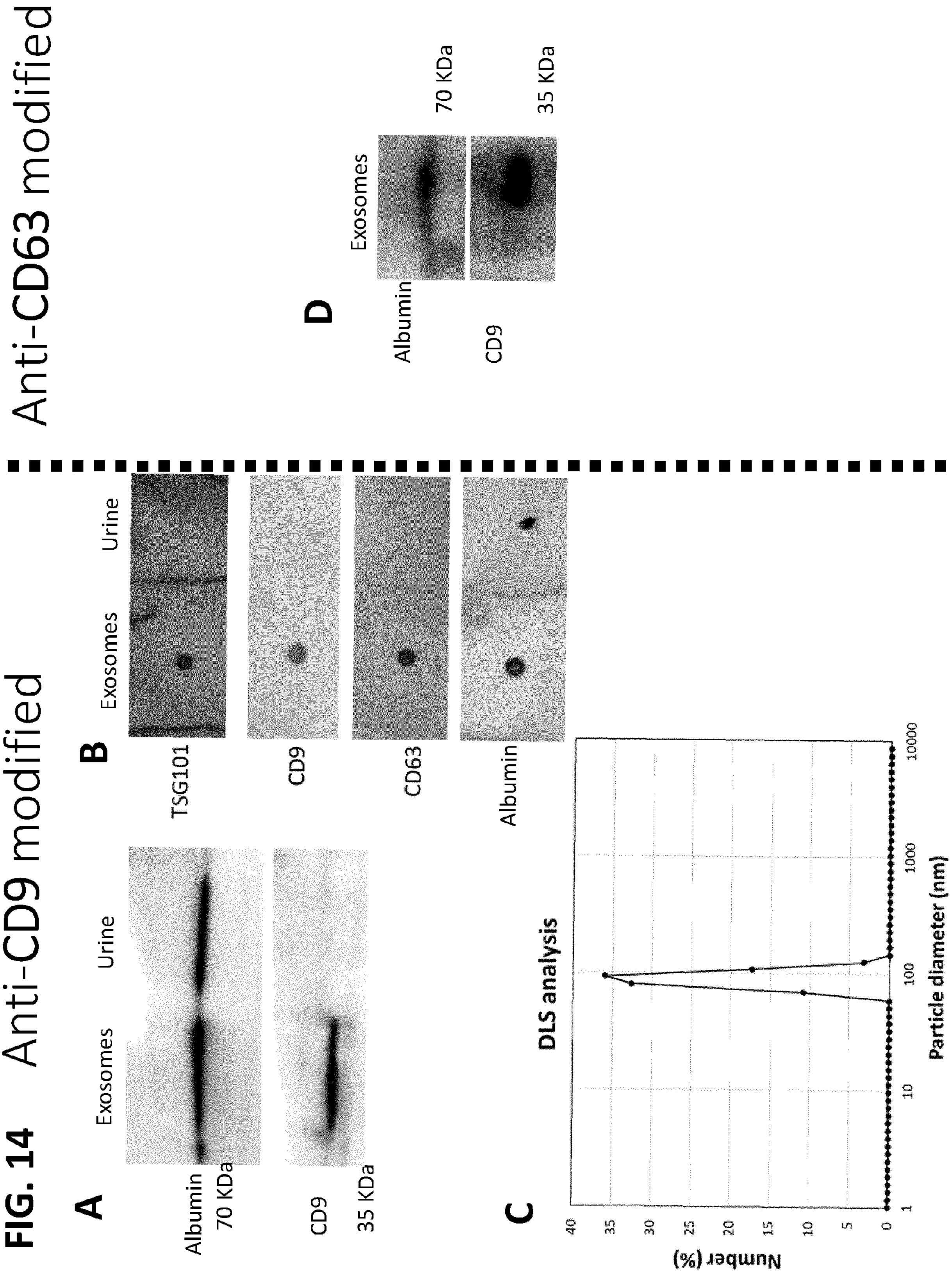
Anti-CD9 modified
A
Exosomes
Urine
Albumin
70 KDa
CD9
35 KDa
B
Exosomes
Urine
TSG101
CD9
CD63
Albumin
C
DLS analysis
Number (%)
Particle diameter (nm)
0
5
10
15
20
25
30
35
40
1
10
100
1000
10000
Anti-CD63 modified
D
Exosomes
Albumin
70 KDa
CD9
35 KDa

FIG. 15

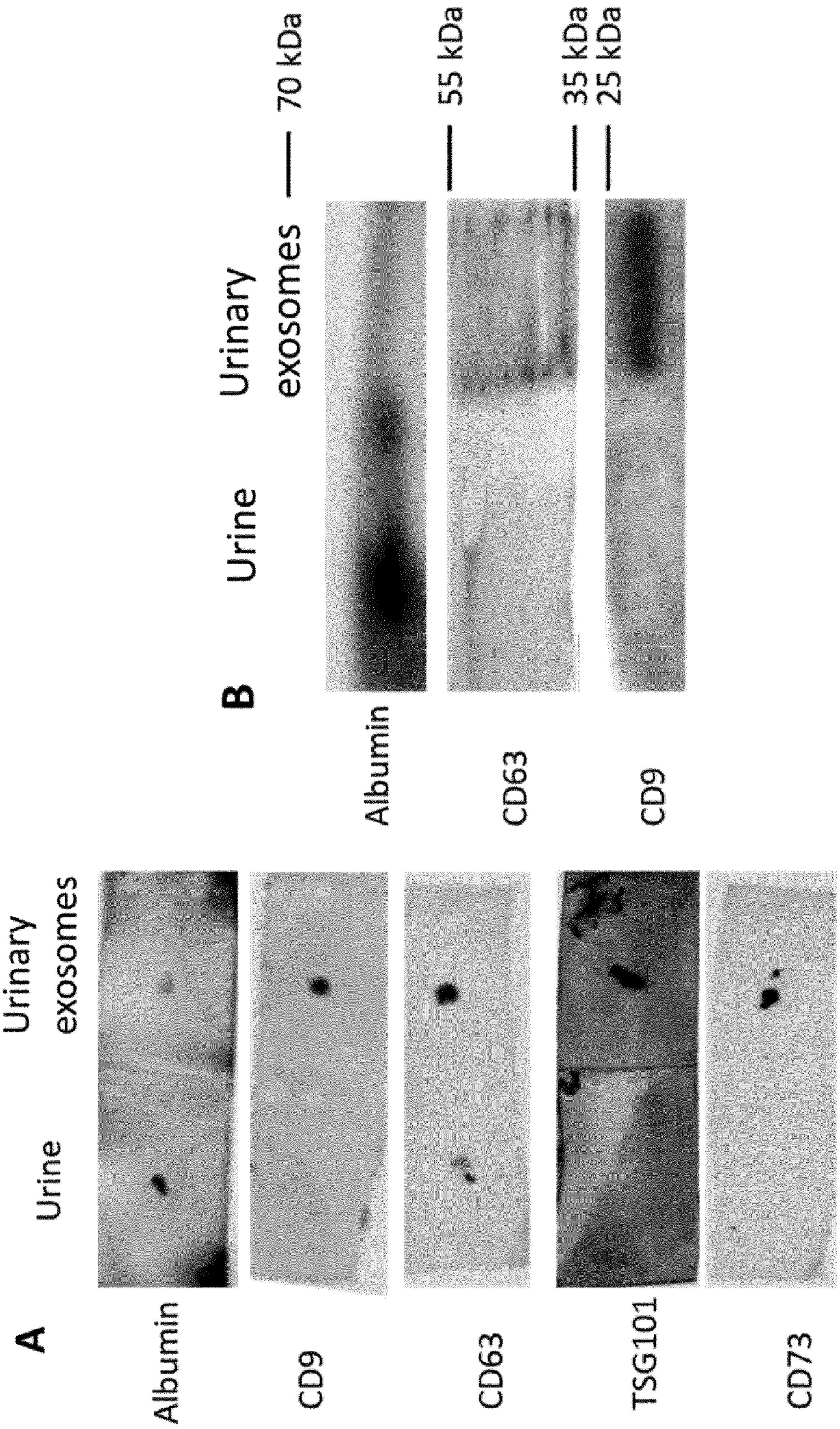
A
Urine
Urinary exosomes
Albumin
CD9
CD63
TSG101
CD73
B
Urine
Urinary exosomes
70 kDa
55 kDa
35 kDa
25 kDa
Albumin
CD63
CD9

FIG. 16

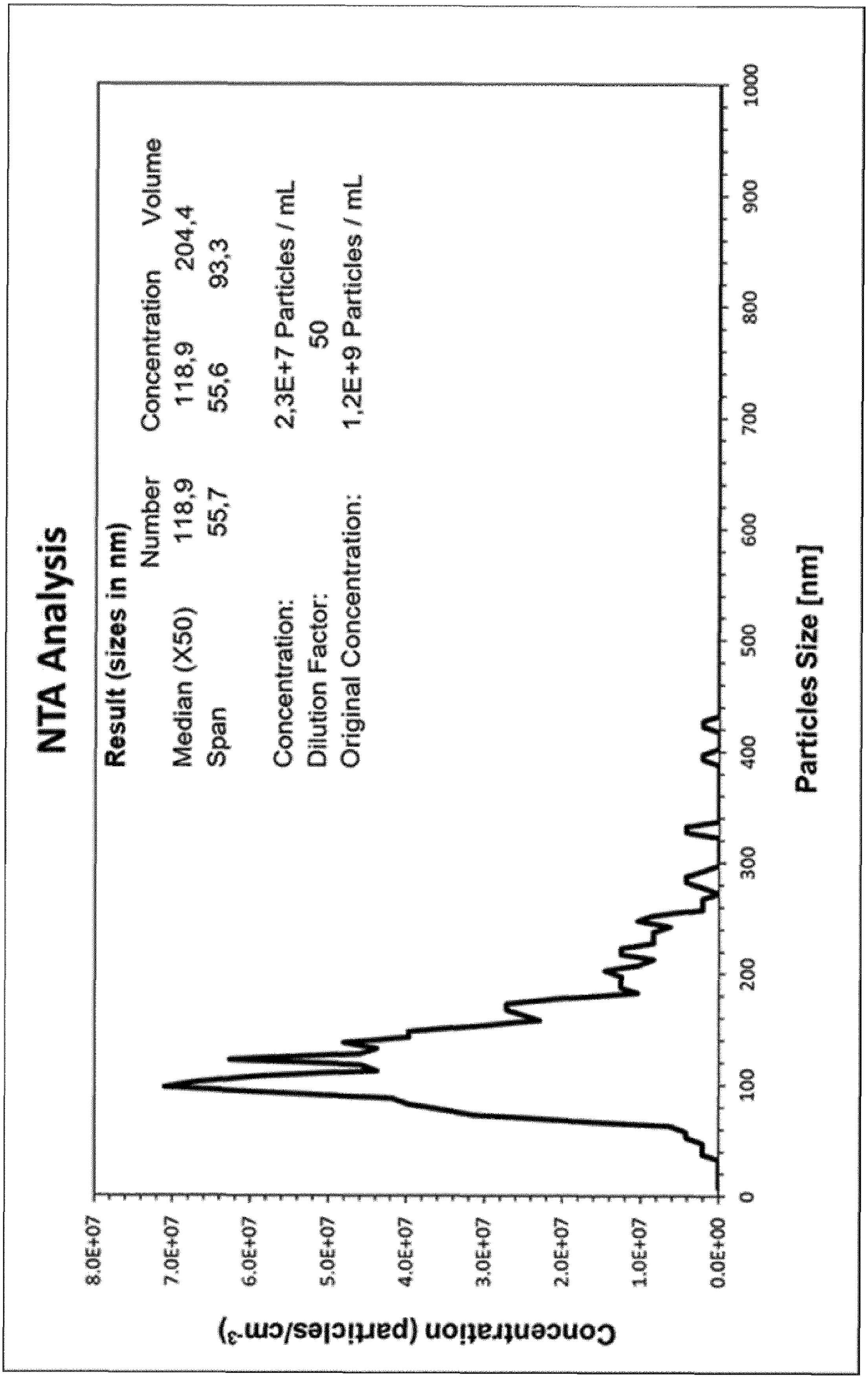
NTA Analysis
Result (sizes in nm)
Number Concentration Volume
Median (X50) 118,9 118,9 204,4
Span 55,7 55,6 93,3
Concentration: 2,3E+7 Particles / mL
Dilution Factor: 50
Original Concentration: 1,2E+9 Particles / mL
Concentration (particles/cm-3)
8.0E+07
7.0E+07
6.0E+07
5.0E+07
4.0E+07
3.0E+07
2.0E+07
1.0E+07
0.0E+00
0
100
200
300
400
500
600
700
800
900
1000
Particles Size [nm]

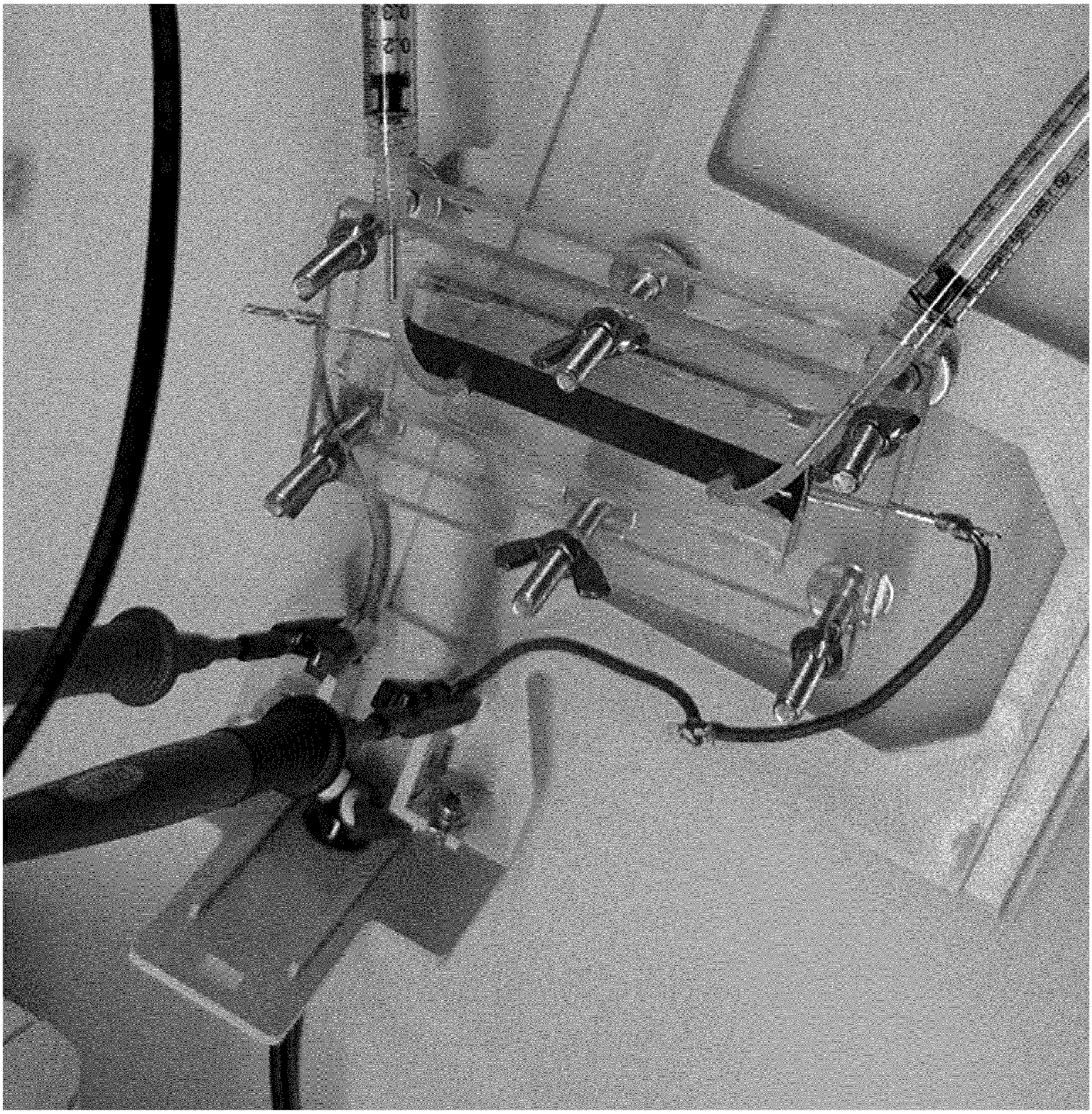

FIG. 17

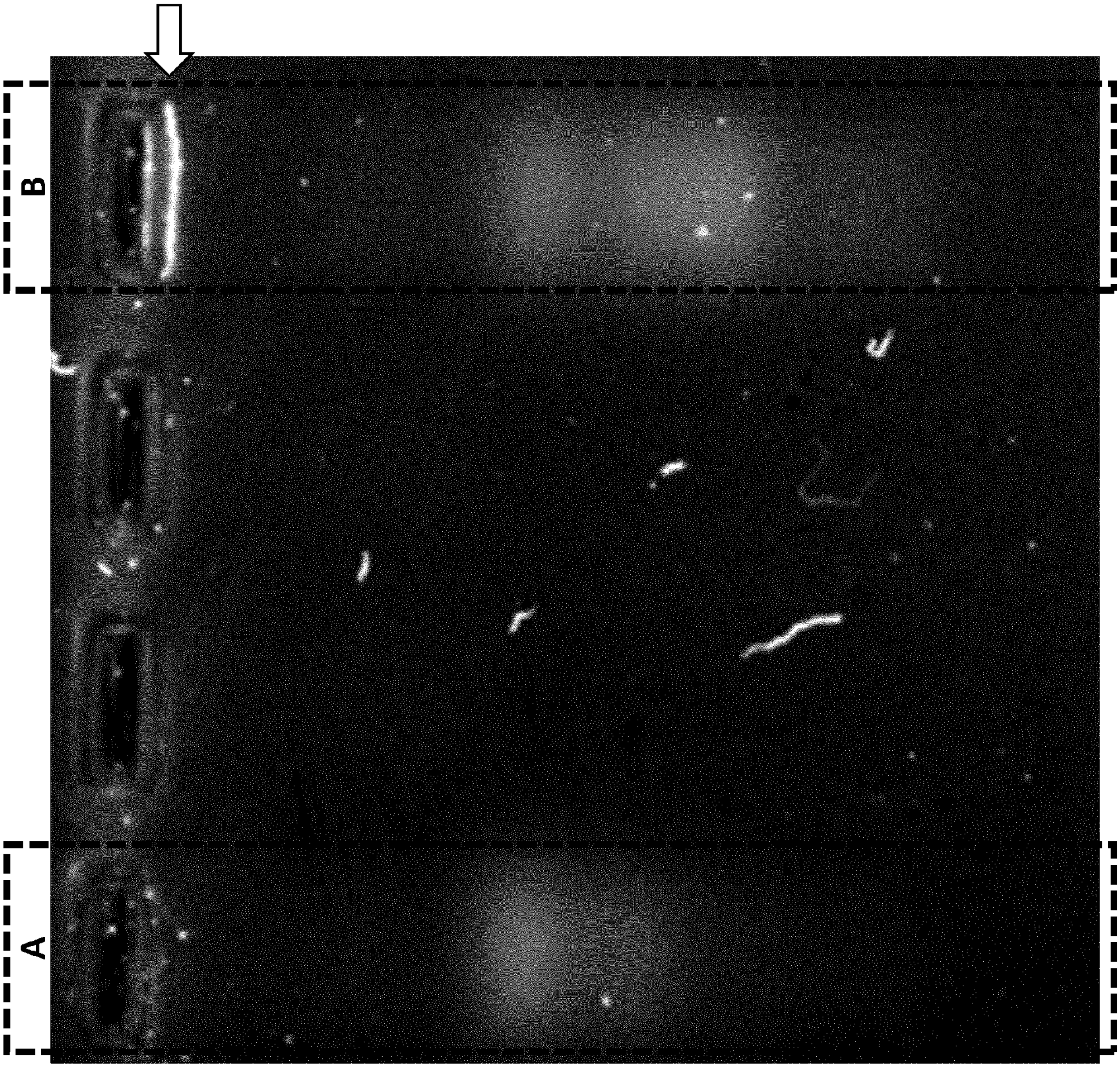


FIG. 18

# FIG. 19

**Result (sizes in nm)**

| | Number | Concentration | Volume |
|---|---|---|---|
| Median (X50) | 154,0 | 154,0 | 332,6 |
| Span | 94,5 | 94,3 | 119,3 |

Concentration: 1,4E+7 Particles / mL
Dilution Factor: 50
Original Concentration: 6,9E+8 Particles / mL

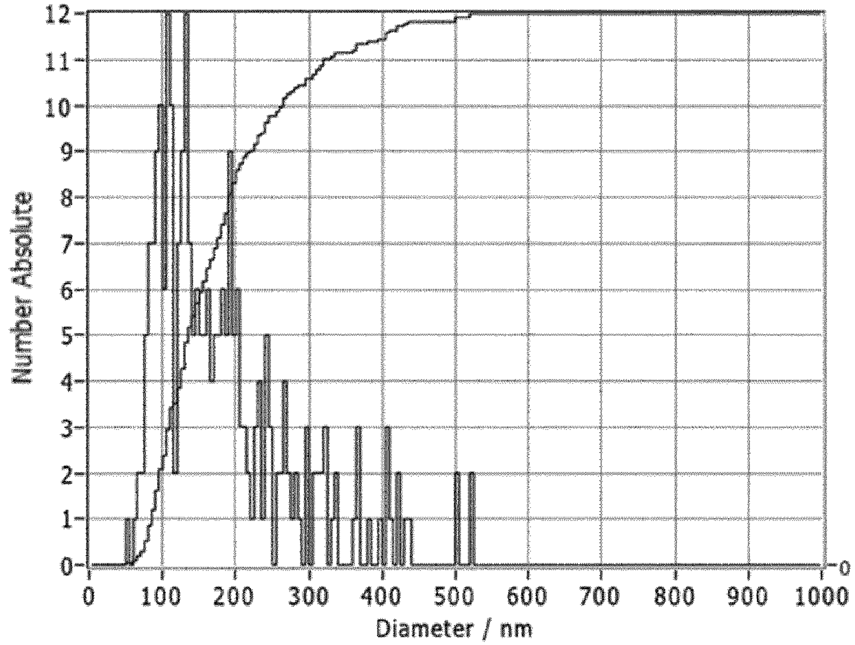


**Result (sizes in nm)**

| | Number | Concentration | Volume |
|---|---|---|---|
| Median (X50) | 126,7 | 126,7 | 209,0 |
| Span | 58,7 | 58,6 | 86,7 |

Concentration: 2,4E+7 Particles / mL
Dilution Factor: 50
Original Concentration: 1,2E+9 Particles / mL

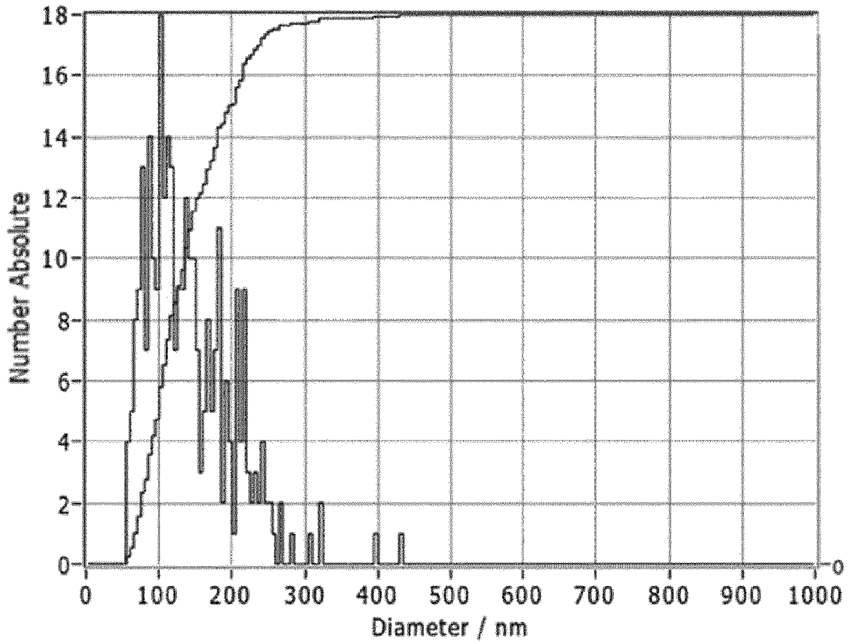

FIG. 20

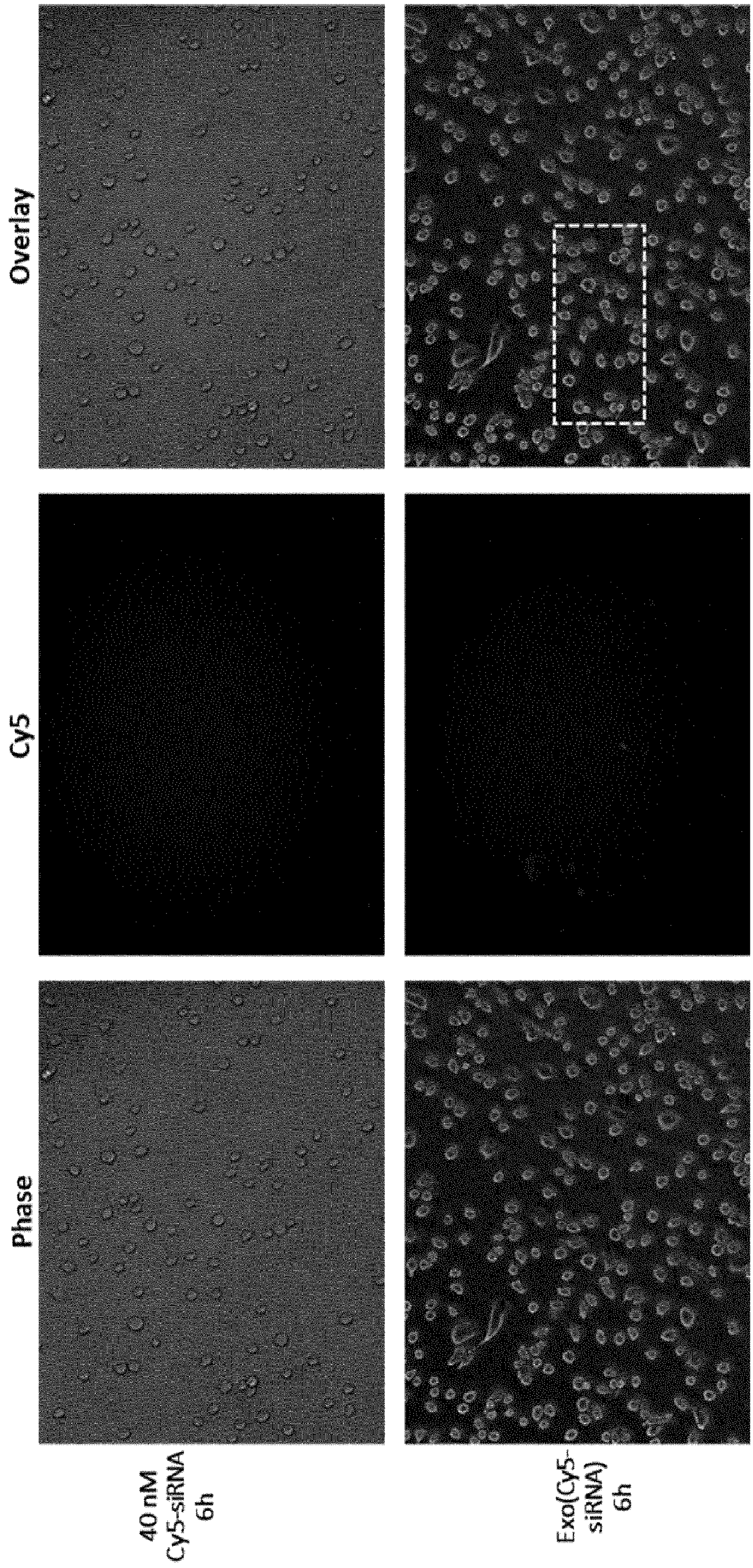

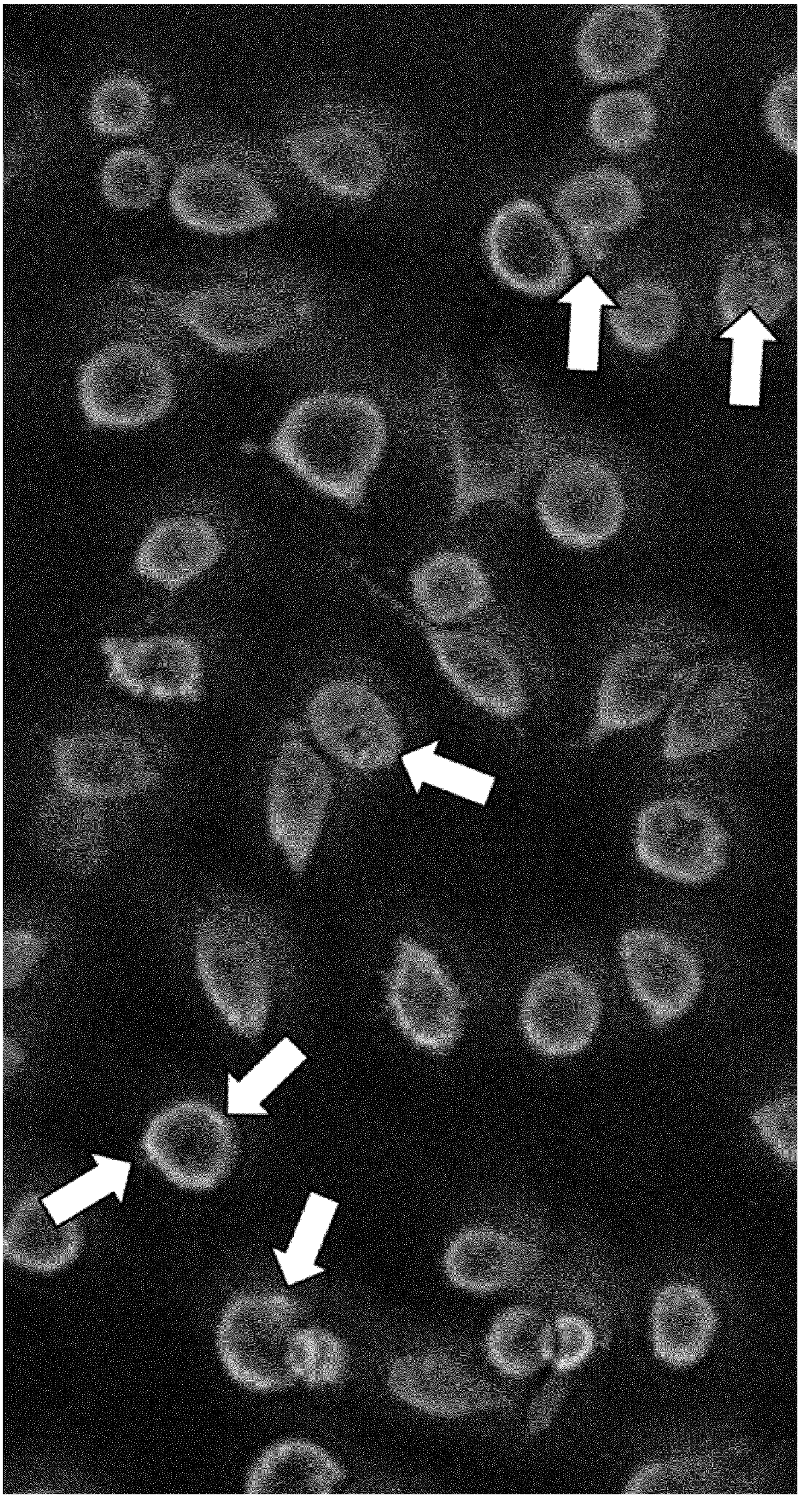

FIG. 21

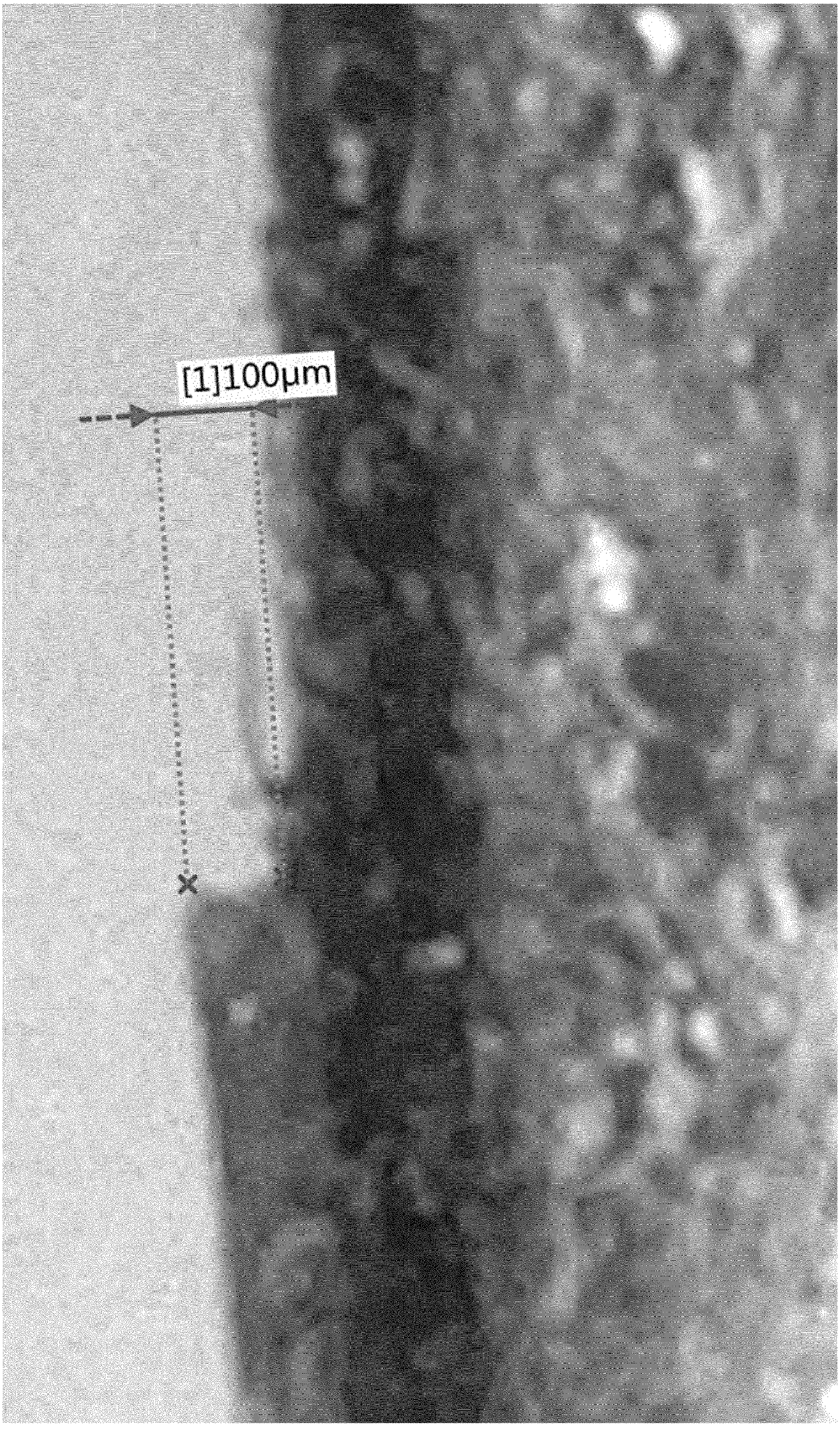


FIG. 22

Europäisches Patentamt
European Patent Office
Office européen des brevets

# EUROPEAN SEARCH REPORT

Application Number
EP 21 20 1750

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2012/162563 A2 (UNIV CALIFORNIA [US]; WONG DAVID T [US]; WEI FANG [US]; LIAO WEI [US]) 29 November 2012 (2012-11-29)<br>* claims 1-46; example 3 *<br>----- | 1-15 | INV.<br>C12N15/82<br>G01N33/543 |
| A | ADAMO ANDREA ET AL: "Flow-Through Comb Electroporation Device for Delivery of Macromolecules",<br>ANALYTICAL CHEMISTRY,<br>vol. 85, no. 3,<br>5 February 2013 (2013-02-05), pages 1637-1641, XP055903314,<br>US<br>ISSN: 0003-2700, DOI: 10.1021/ac302887a<br>Retrieved from the Internet:<br>URL:https://pubs.acs.org/doi/pdf/10.1021/ac302887a><br>* abstract; figure 1 *<br>----- | 1-15 | |
| X | EP 2 940 120 A1 (LONZA COLOGNE GMBH [DE]) 4 November 2015 (2015-11-04)<br>* Paragraphs 26, 47, 53;<br>claims 1-15 *<br>----- | 4-6 | TECHNICAL FIELDS SEARCHED (IPC)<br>C12N<br>G01N |
| A | KASHEFI-KHEYRABADI LEILA ET AL: "Detachable microfluidic device implemented with electrochemical aptasensor (DeMEA) for sequential analysis of cancerous exosomes",<br>BIOSENSORS AND BIOELECTRONICS, ELSEVIER SCIENCE LTD, UK, AMSTERDAM , NL,<br>vol. 169, 17 September 2020 (2020-09-17), XP086287461,<br>ISSN: 0956-5663, DOI: 10.1016/J.BIOS.2020.112622<br>[retrieved on 2020-09-17]<br>* abstract; figures 1, 3 *<br>----- | 1-15 | |

The present search report has been drawn up for all claims

2

| Place of search | Date of completion of the search | Examiner |
|---|---|---|
| Munich | 21 March 2022 | Moreno de Vega, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 20 1750

This annex lists the patent family members relating to the patent documents cited in the above-mentioned European search report. The members are as contained in the European Patent Office EDP file on
The European Patent Office is in no way liable for these particulars which are merely given for the purpose of information.

21-03-2022

| Patent document cited in search report | Publication date | Patent family member(s) | Publication date |
|---|---|---|---|
| WO 2012162563 A2 | 29-11-2012 | EP 2715351 A2 | 09-04-2014 |
| | | US 2014315195 A1 | 23-10-2014 |
| | | US 2019078159 A1 | 14-03-2019 |
| | | WO 2012162563 A2 | 29-11-2012 |
| EP 2940120 A1 | 04-11-2015 | AU 2015254739 A1 | 17-11-2016 |
| | | AU 2015254741 A1 | 17-11-2016 |
| | | AU 2018205130 A1 | 26-07-2018 |
| | | CA 2946472 A1 | 05-11-2015 |
| | | CA 2946666 A1 | 05-11-2015 |
| | | CN 106255746 A | 21-12-2016 |
| | | CN 106459872 A | 22-02-2017 |
| | | CN 111321077 A | 23-06-2020 |
| | | DK 2940120 T3 | 26-11-2018 |
| | | DK 2940121 T3 | 26-11-2018 |
| | | DK 3260163 T3 | 02-09-2019 |
| | | EP 2940120 A1 | 04-11-2015 |
| | | EP 2940121 A1 | 04-11-2015 |
| | | EP 3260163 A1 | 27-12-2017 |
| | | ES 2692530 T3 | 04-12-2018 |
| | | ES 2693752 T3 | 13-12-2018 |
| | | ES 2751732 T3 | 01-04-2020 |
| | | JP 6642875 B2 | 12-02-2020 |
| | | JP 6750810 B2 | 02-09-2020 |
| | | JP 2017514474 A | 08-06-2017 |
| | | JP 2017514475 A | 08-06-2017 |
| | | JP 2019213539 A | 19-12-2019 |
| | | KR 20170017876 A | 15-02-2017 |
| | | KR 20170017899 A | 15-02-2017 |
| | | US 2017233715 A1 | 17-08-2017 |
| | | US 2017233716 A1 | 17-08-2017 |
| | | US 2019144844 A1 | 16-05-2019 |
| | | US 2020325466 A1 | 15-10-2020 |
| | | WO 2015165879 A1 | 05-11-2015 |
| | | WO 2015165881 A1 | 05-11-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8901284 B2
- US 20130337440 A
- US 20130273544 A
- US 20130052647 A1
- US 10948451 B2
- US 2012142611 A
- US 2016250341 A
- US 2016075767 A
- US 2015368302 A


### Non-patent literature cited in the description


- **SAMBROOK et al.** Molecular Cloning: A Laboratory Manual. Cold Spring Harbor Laboratory Press, 2012
- **AUSUBEL et al.** Short Protocols in Molecular Biology. John Wiley & Sons, Inc, 2002
- **SKERRA.** *Biochim. Biophys. Acta,* 2000, vol. 1482 (1-2), 337-50
- **KWAN et al.** *Structure,* 2003, vol. 11 (7), 803-813
- **FAN et al.** *Nano Lett,* 2010, vol. 10, 3823-3827
- **MANNELI et al.** *Langmuir,* 2016, vol. 32 (48), 12632-12640
- **STEENO et al.** *Chem. Mater.,* 2020, vol. 32 (12), 5246-5255
- **ZHANG et al.** *J. Am. Chem. Soc.,* 2019, vol. 141 (20), 8277-8288
- **NAYAK et al.** Carbon-Containing Polymer Composites. Springer, 2018, 65-98
- **KHAN ; NISHINA.** *Nanoscale,* 2021, vol. 2021 (13), 36-50
- **DENNLER et al.** *Antibodies,* 2015, vol. 4 (3), 197-224